(19) Europäisches Patentamt
European Patent Office
Office européen des brevets

(11) Veröffentlichungsnummer: **0 192 951 A2**

# (12) EUROPÄISCHE PATENTANMELDUNG

(21) Anmeldenummer: **86100722.7**

(22) Anmeldetag: **21.01.86**

(51) Int. Cl.⁴: **C 07 D 231/42**
**A 01 N 43/56**

(30) Priorität: **02.02.85 DE 3503609**

(43) Veröffentlichungstag der Anmeldung:
**03.09.86 Patentblatt 86/36**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Schallner, Otto, Dr.**
**Noldeweg 22**
**D-4019 Monheim(DE)**

(72) Erfinder: **Gehring, Reinhold, Dr.**
**Dasnöckel 49**
**D-5600 Wuppertal 11(DE)**

(72) Erfinder: **Stetter, Jörg, Dr.**
**Gellertweg 4**
**D-5600 Wuppertal 1(DE)**

(72) Erfinder: **Santel, Hans-Joachim, Dr.**
**Grünstrasse 9a**
**D-5090 Leverkusen 1(DE)**

(72) Erfinder: **Schmidt, Robert R., Dr.**
**Im Waldwinkel 110**
**D-5060 Bergisch Gladbach 2(DE)**

(54) **5-Sulfonamido-1-aryl-pyrazole.**

(57) 5-Sulfonamido-1-aryl-pyrazole der Formel (I),

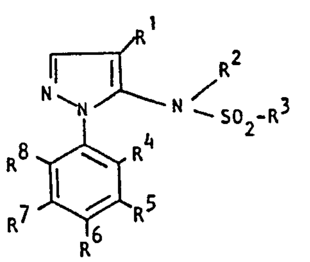

(I)

in welcher

$R^1$ für Wasserstoff, Nitro, Nitroso oder Halogen steht,

$R^2$ für Wasserstoff, Alkyl, für einen Rest $R^3-SO_2-$ oder für ein salzartig gebundenes organisches oder anorganisches Kation steht,

$R^3$ für Alkyl, Alkenyl, Cycloalkyl, Halogenalkyl, Hydroxyalkyl, Alkoxyalkyl oder für jeweils gegebenenfalls substituiertes Aryl steht,

$R^4$ und $R^6$ unabhängig voneinander für Cyano, Nitro, Halogen, Alkyl, Alkoxy, Alkoxycarbonyl, Halogenalkyl, Halogenalkoxy oder für einen Rest $-S(O)_n-R^9$ stehen und

$R^5$, $R^7$ und $R^8$ unabhängig voneinander und von $R^4$ und $R^6$ für die gleichen Reste wie $R^4$ und $R^6$ stehen und zusätzlich für Wasserstoff stehen, wobei

$R^9$ für Alkyl, Halogenalkyl, Amino, Alkylamino oder Dialkylamino steht und

n für eine Zahl 0, 1 oder 2 steht,

Verfahren zu ihrer Herstellung und ihre Verwendung als Hervizide.

EP 0 192 951 A2

0192951

BAYER AKTIENGESELLSCHAFT 5090 Leverkusen, Bayerwerk

Konzernverwaltung RP
Patentabteilung KM/Ke-c

Ib

# 5-Sulfonamido-1-aryl-pyrazole

Die Erfindung betrifft neue 5-Sulfonamido-1-aryl-pyrazole, mehrere Verfahren zu ihrer Herstellung und ihre Verwendung als Herbizide.

Es ist bereits bekannt, daß bestimmte 1-Aryl-pyrazole, wie beispielsweise das 4-Cyano-5-propionamido-1-(2,3,4-trichlorphenyl)-pyrazol, herbizide insbesondere auch selektiv-herbizide Eigenschaften besitzen (vgl. z.B. DE-OS 3 226 513).

Die herbizide Wirksamkeit dieser vorbekannten 1-Aryl-pyrazole gegenüber Schadpflanzen ist jedoch ebenso wie ihre Verträglichkeit gegenüber wichtigen Kulturpflanzen nicht immer in allen Anwendungsbereichen völlig zufriedenstellend.

Es wurden neue 5-Sulfonamido-1-aryl-pyrazole der allgemeinen Formel (I),

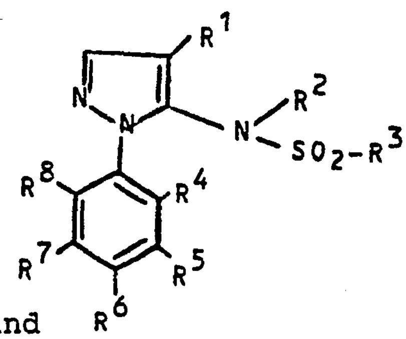

(I)

in welcher

$R^1$ für Wasserstoff, Nitro, Nitroso oder Halogen steht,

$R^2$ für Wasserstoff, Alkyl, für einen Rest $R^3-SO_2-$ oder für ein salzartig gebundenes anorganisches oder organisches Kation steht,

$R^3$ für Alkyl, Alkenyl, Cycloalkyl, Halogenalkyl, Hydroxyalkyl, Alkoxyalkyl oder für jeweils gegebenenfalls substituiertes Aryl steht,

$R^4$ und $R^6$ unabhängig voneinander für Cyano, Nitro, Halogen, Alkyl, Alkoxy, Alkoxycarbonyl, Halogenalkyl, Halogenalkoxy oder für einen Rest $-S(O)_n-R^9$ stehen und

$R^5$, $R^7$ und $R^8$ unabhängig voneinander und von $R^4$ und $R^6$ für die gleichen Reste wie $R^4$ und $R^6$ stehen und zusätzlich für Wasserstoff stehen, wobei

$R^9$ für Alkyl, Halogenalkyl, Amino, Alkylamino oder Dialkylamino steht und

n für eine Zahl 0, 1 oder 2 steht,

gefunden.

Weiterhin wurde gefunden, daß man die neuen 5-Sulfonamido-1-aryl-pyrazole der allgemeinen Formel (I),

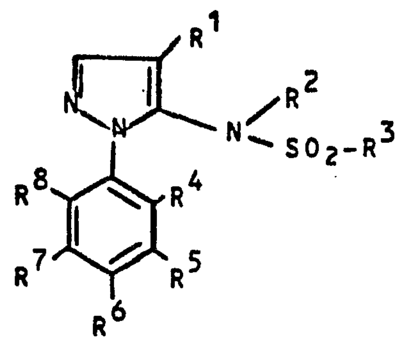

(I)

in welcher

$R^1$ für Wasserstoff, Nitro, Nitroso oder Halogen steht,

$R^2$ für Wasserstoff, Alkyl, für einen Rest $R^3-SO_2-$ oder für ein salzartig gebundenes anorganisches oder organisches Kation steht,

$R^3$ für Alkyl, Alkenyl, Cycloalkyl, Halogenalkyl, Hydroxyalkyl, Alkoxyalkyl oder für jeweils gegebenenfalls substituiertes Aryl steht,

$R^4$ und $R^6$ unabhängig voneinander für Cyano, Nitro, Halogen, Alkyl, Alkoxy, Alkoxycarbonyl, Halogenalkyl, Halogenalkoxy oder für einen Rest $-S(O)_n-R^9$ stehen und

$R^5$, $R^7$ und $R^8$ unabhängig voneinander und von $R^4$ und $R^6$ für die gleichen Reste wie $R^4$ und $R^6$ stehen und zusätzlich für Wasserstoff stehen, wobei

$R^9$ für Alkyl, Halogenalkyl, Amino, Alkylamino oder Dialkylamino steht und

n für eine Zahl 0, 1 oder 2 steht,

erhält, wenn man

(a) 5-Aminopyrazole der Formel (II),

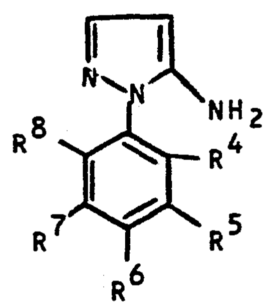

(II)

in welcher

$R^4$, $R^5$, $R^6$, $R^7$ und $R^8$ die oben angegebene Bedeutung haben,

mit Sulfonylchloriden der Formel (III),

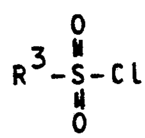

(III)

in welcher

$R^3$ die oben angegebene Bedeutung hat,

gegebenenfalls in Gegenwart eines Verdünnungsmittels und gegebenenfalls in Gegenwart eines Säurebindemittels umsetzt,

oder wenn man

(b) die nach Verfahren (a) erhältlichen Bisulfonylamine der Formel (Ia),

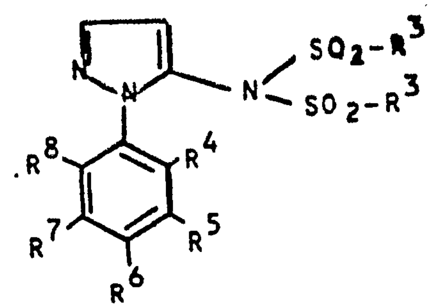

(Ia)

in welcher

$R^3$, $R^4$, $R^5$, $R^6$, $R^7$ und $R^8$ die oben angegebene Bedeutung haben

mit Basen gegebenenfalls in Gegenwart eines Verdünnungsmittels spaltet,

oder wenn man

(c) die nach Verfahren (a) oder (b) erhältlichen 5-Sulfonamido-pyrazole der Formel (Ib),

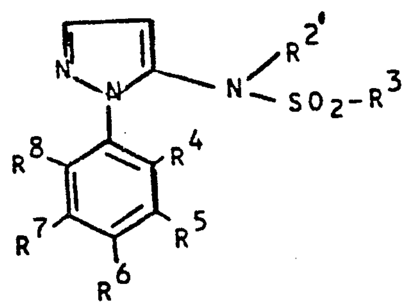

(Ib)

in welcher

$R^{2'}$ für Wasserstoff oder für einen Rest $R^3$-$SO_2$- steht und

$R^3$, $R^4$, $R^5$, $R^6$, $R^7$ und $R^8$ die oben angegebene Bedeutung haben,

mit elektrophilen Agenzien der Formel (IV),

$$R^{1'}\text{-E} \qquad \text{(IV)}$$

in welcher

$R^{1'}$ für Halogen, Nitroso oder Nitro steht und

E für eine elektronenanziehende Abgangsgruppierung steht,

gegebenenfalls in Gegenwart eines Verdünnungsmittels und gegebenenfalls in Gegenwart eines Katalysators oder Reaktionshilfsmittels in 4-Stellung substituiert,

oder wenn man

(d) die nach Verfahren (a), (b) oder (c) erhältlichen 5-Sulfonamido-pyrazole der Formel (Ic),

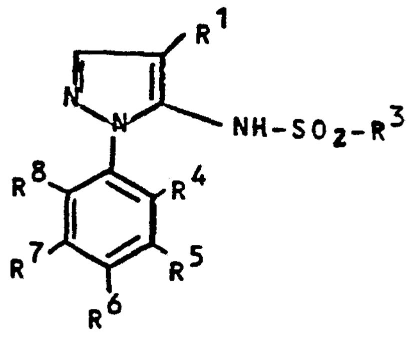

(Ic)

0192951

in welcher

$R^1$, $R^3$, $R^4$, $R^5$, $R^6$, $R^7$ und $R^8$ die oben angegebene Bedeutung haben,

entweder

(α) mit Alkylierungsmitteln der Formel (V),

$$R^{2''}-E' \qquad (V)$$

in welcher

$R^{2''}$ für Alkyl steht und
$E'$ für eine elektronenanziehende Abgangsgruppe steht,

gegebenenfalls in Gegenwart eines Verdünnungsmittels und gegebenenfalls in Gegenwart eines Säurebindemittels bzw. Katalysators am Stickstoffatom der Sulfonamidogruppe alkyliert,

oder

(ß) mit Verbindungen der Formel (VI),

$$M^{\oplus} G^{\ominus} \qquad (VI)$$

in welcher

$M^{\oplus}$ für ein Äquivalent eines anorganischen oder organischen Kations steht und

$G^{\ominus}$ für ein Äquivalent eines geeigneten Gegenions steht,

umsetzt oder mit primären, sekundären oder tertiären Aminen gegebenenfalls in Gegenwart eines Verdünnungsmittels am Stickstoff der Sulfonamidogruppe ein Salz bildet.

Schließlich wurde gefunden, daß die neuen 5-Sulfonamido-1-aryl-pyrazole der allgemeinen Formel (I) herbizide, insbesondere auch selektiv-herbizide Eigenschaften besitzen.

Überraschenderweise zeigen die erfindungsgemäßen 5-Sulfonamido-1-aryl-pyrazole der allgemeinen Formel (I) neben einer deutlich verbesserten allgemein-herbiziden Wirksamkeit gegenüber Schadpflanzen eine erheblich verbesserte Verträglichkeit gegenüber wichtigen Kulturpflanzen als die aus dem Stand der Technik bekannten 1-Aryl-pyrazole, wie beispielsweise das 4-Cyano-5-propionylamino-1-(2,3,4-trichlorphenyl)-pyrazol, welches eine chemisch und wirkungsmäßig naheliegende Verbindung ist.

Die erfindungsgemäßen 5-Sulfonamido-1-aryl-pyrazole sind durch die Formel (I) allgemein definiert. Bevorzugt sind Verbindungen der Formel (I), bei welchen

$R^1$ für Wasserstoff, Nitro, Nitroso oder Halogen steht,

$R^2$ für Wasserstoff, geradkettiges oder verzweigtes Alkyl mit 1 bis 8 Kohlenstoffatomen, für einen Rest $R^3$-$SO_2$- oder für ein Äquivalent eines Alkali- oder Erdalkali- oder Übergangsmetallkations oder für ein gegebenenfalls substituiertes Ammoniumion steht,

$R^3$ für jeweils geradkettiges oder verzweigtes Alkyl, Alkenyl, Halogenalkyl, Alkoxyalkyl oder Hydroxyalkyl mit jeweils bis zu 4 Kohlenstoffatomen und gegebenenfalls bis zu 9 gleichen oder verschiedenen Halogenatomen, für Cycloalkyl mit 3 bis 7 Kohlenstoffatomen oder für jeweils gegebenenfalls einfach oder mehrfach, gleich oder verschieden durch Halogen, Cyano, Nitro sowie jeweils niederes Alkyl, Alkoxy, Alkylthio oder Halogenalkyl substituiertes Benzyl oder Phenyl steht,

$R^4$ und $R^6$ unabhängig voneinander für Cyano, Halogen oder für jeweils geradkettiges oder verzweigtes Alkyl, Alkoxy oder Alkoxycarbonyl mit jeweils bis zu 4 Kohlenstoffatomen, außerdem für jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder für einen Rest $-S(O)_n-R^9$ stehen, und

$R^5$, $R^7$ und $R^8$ unabhängig voneinander und von $R^4$ und $R^6$ für die gleichen Reste wie $R^4$ und $R^6$ stehen und zusätzlich für Wasserstoff stehen, wobei

$R^9$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

n für eine Zahl 0, 1 oder 2 steht.

Besonders bevorzugt sind Verbindungen der Formel (I), bei welchen

$R^1$ für Wasserstoff, Nitro, Nitroso, Fluor, Chlor, Brom oder Iod steht,

$R^2$ für Wasserstoff, Methyl, Ethyl, n- oder i-Propyl, n-, i-, s- oder t-Butyl, für einen Rest $R^3$-$SO_2$- oder für ein Äquivalent eines Natrium-, Kalium-, Magnesium-, Calcium-, Barium-, Kupfer-, Zink-, Mangan-, Zinn-, Eisen-, Cobalt- oder Nickelions steht, oder für ein gegebenenfalls ein- bis dreifach gleich oder verschieden durch Methyl, Ethyl, n- oder i-Propyl, n-, i-, s- oder t-Butyl oder Phenyl substituiertes Ammoniumion steht,

$R^3$ für Methyl, Ethyl, n- oder i-Propyl, n-, i-, s- oder t-Butyl, Allyl, Butenyl, Methoxymethyl, Methoxyethyl, Hydroxymethyl, Hydroxyethyl, Chlormethyl,

Dichlormethyl, Trifluormethyl, Cyclopropyl, Cyclopentyl, Cyclohexyl oder für jeweils gegebenenfalls ein- bis dreifach gleich oder verschieden substituiertes Benzyl oder Phenyl steht, wobei als Substituenten infrage kommen:
Fluor, Chlor, Brom, Cyano, Nitro, Methyl, Ethyl, Methoxy, Methylthio oder Trifluormethyl,

$R^4$ und $R^6$ unabhängig voneinander für Cyano, Nitro, Fluor, Chlor, Brom, Iod, Methyl, Ethyl, n- und i-Propyl, n-, i-, s- und t-Butyl, für Methoxy, Ethoxy, Methoxycarbonyl, Ethoxycarbonyl, für Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, für Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder für einen Rest $-S(O)_n-R^9$ stehen und

$R^5$, $R^7$ und $R^8$ unabhängig voneinander und von $R^4$ und $R^6$ für die gleichen Reste wie $R^4$ und $R^6$ stehen und zusätzlich für Wasserstoff stehen, wobei

$R^9$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht und

n für eine Zahl 0, 1 oder 2 steht.

Im einzelnen seien außer den bei den Herstellungsbeispielen genannten Verbindungen die in der folgenden Tabelle aufgeführten 5-Sulfonamido-1-aryl-pyrazole der allgemeinen Formel (I) genannt:

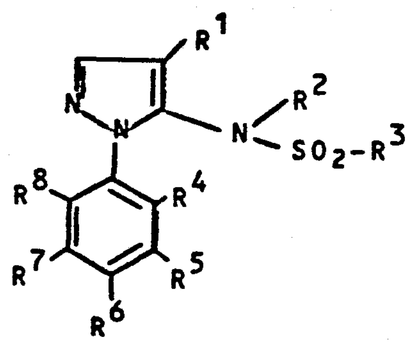

(I)

Tabelle 1

| $R^1$ | $R^2$ | $R^3$ | $R^4$ | $R^5$ | $R^6$ | $R^7$ | $R^8$ |
|---|---|---|---|---|---|---|---|
| H | H | $CH_3$ | Cl | H | Cl | H | Cl |
| H | H | 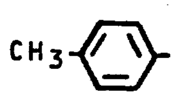 | Cl | H | Cl | H | Cl |
| H | H | 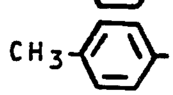 | Cl | H | $CF_3$ | H | Cl |
| H | H | $CH_3$ | Cl | Cl | Cl | H | H |
| H | H | $CH_3$ | Cl | H | $CF_3$ | H | Br |
| H | $CH_3$ | $CH_3$ | Cl | Cl | $CF_3$ | H | Cl |

Tabelle 1 (Fortsetzung)

| $R^1$ | $R^2$ | $R^3$ | $R^4$ | $R^5$ | $R^6$ | $R^7$ | $R^8$ |
|---|---|---|---|---|---|---|---|
| H | H | $CH_3$ | Cl | F | $CF_3$ | Cl | Cl |
| H | H | $CH_3$ | Cl | F | $CF_3$ | F | F |
| H | H | $CH_3$ | F | F | $CF_3$ | F | F |
| H | H | $CH_3$ | Cl | F | Cl | F | Cl |
| H | H | $C_2H_5$ | Cl | H | Br | H | Cl |
| H | H | $CH_3$ | F | F | F | F | F |
| H | $C_2H_5$ | $CH_2Cl$ | Cl | H | $CF_3$ | H | H |
| H | $(CH_2)_2OH$ | $CF_3$ | Cl | Cl | Cl | H | H |
| H | H | $CH_3$ | Cl | H | $OCF_3$ | H | Cl |
| H | H | $C_4H_9$ | Cl | H | $SCF_3$ | H | Cl |
| H | $CH_3$ | $CH_3$ | Cl | Cl | $OCF_3$ | H | Cl |
| H | H | $(CH_2)_2Cl$ | Cl | Cl | $SCF_3$ | H | Cl |
| H | H | $CF_3$ | Cl | Cl | Cl | H | Cl |
| H | H | $CF_3$ | Cl | H | Cl | H | Cl |
| H | $CH(CH_3)_2$ | $CH_3$ | Br | H | Br | H | Br |
| H | H | $C_4F_9$ | Br | H | $CH_3$ | H | H |
| H | H | $C_4H_9$ | Cl | H | $OCF_2CHF_2$ | H | H |

Tabelle 1 (Fortsetzung)

| $R^1$ | $R^2$ | $R^3$ | $R^4$ | $R^5$ | $R^6$ | $R^7$ | $R^8$ |
|---|---|---|---|---|---|---|---|
| H | H | $CH_3$ | Cl | H | $SO_2CCl_2F$ | H | Cl |
| H | H | $CH_3$ | Cl | H | $SO_2CClF_2$ | H | Cl |
| H | $CH_3$ | $CH_2Cl$ | Br | H | $SCF_3$ | H | Br |
| H | H | $C_4H_9$ | Br | H | $SCF_3$ | H | H |
| H | H | $CH_3$ | $CF_3$ | F | Cl | H | Cl |
| H | $CH_3$ | $CH_3$ | $CF_3$ | H | Cl | H | H |
| H | H | $C_4H_9$ | $CF_3$ | H | Br | H | H |
| H | H | $CH_3$ | Cl | F | CN | F | Cl |
| H | H | $CH_3$ | F | F | CN | F | F |
| H | H | $CH_3$ | F | Cl | F | Cl | F |
| H | H | 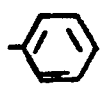 | Cl | Cl | $CF_3$ | H | Cl |
| H | $CH_3$ | 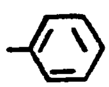 | Cl | H | $OCF_3$ | H | H |
| H | $CH_3$ | 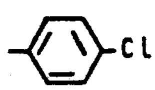 | Cl | H | $CF_3$ | H | Cl |
| H | H | 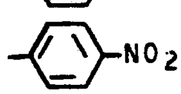 | Cl | H | $CF_3$ | H | Cl |
| H | $CH_3$ | $CH_2Cl$ | Cl | H | $CF_3$ | H | H |
| H | $C_3H_7$ | $CH_3$ | Cl | Cl | $CF_3$ | H | Cl |
| H | $(CH_2)_2OH$ | $C_4H_9$ | Cl | H | $SO_2CF_3$ | H | Cl |

Tabelle 1 (Fortsetzung)

| $R^1$ | $R^2$ | $R^3$ | $R^4$ | $R^5$ | $R^6$ | $R^7$ | $R^8$ |
|---|---|---|---|---|---|---|---|
| H | H | $CH_3$ | Cl | H | $SO_2CF_3$ | H | H |
| H | H | $CH_3$ | Cl | H | $SOCF_3$ | H | H |
| H | H | $CH_3$ | Cl | Cl | Cl | H | Cl |
| H | H | $CF_3$ | Cl | H | $OCF_3$ | H | Cl |
| H | $CH_3$ | $CF_3$ | Cl | Cl | Cl | H | H |
| H | H | $C_4F_9$ | Cl | Cl | $CF_3$ | H | Cl |
| H | $CH_3$ | $C_4F_9$ | Cl | H | $SCF_3$ | H | Cl |
| H | H | $CH_3$ | $CF_3$ | H | $CF_3$ | H | H |
| H | H | $CH_3$ | Cl | H | $NO_2$ | H | Cl |
| H | H | $CH_3$ | $NO_2$ | H | $NO_2$ | H | H |
| H | H | $CH_3$ | Cl | H | $CH_3$ | H | Cl |
| H | H | $CF_3$ | Cl | H | $CH(CH_3)_2$ | H | Cl |
| H | H | $CH_2Cl$ | Cl | H | $C(CH_3)_3$ | H | Cl |
| H | H | $CH_3$ | Br | H | $OCF_3$ | H | Cl |
| NO | H | $CH_3$ | Cl | H | $CF_3$ | H | Br |
| NO | $CH_3$ | $CH_3$ | Cl | Cl | $CF_3$ | H | Cl |
| NO | H | $CH_3$ | Cl | F | $CF_3$ | Cl | Cl |

Tabelle 1 (Fortsetzung)

| $R^1$ | $R^2$ | $R^3$ | $R^4$ | $R^5$ | $R^6$ | $R^7$ | $R^8$ |
|---|---|---|---|---|---|---|---|
| NO | H | $CH_3$ | Cl | F | $CF_3$ | F | F |
| NO | H | $CH_3$ | F | F | $CF_3$ | F | F |
| NO | H | $CH_3$ | Cl | F | Cl | F | Cl |
| NO | H | $C_2H_5$ | Cl | H | Br | H | Cl |
| NO | H | $CH_3$ | F | F | F | F | F |
| NO | $C_2H_5$ | $CH_2Cl$ | Cl | H | $CF_3$ | H | H |
| NO | $(CH_2)_2OH$ | $CF_3$ | Cl | Cl | Cl | H | H |
| NO | H | $CH_3$ | Cl | H | $OCF_3$ | H | Cl |
| NO | H | $C_4H_9$ | Cl | H | $SCF_3$ | H | Cl |
| NO | $CH_3$ | $CH_3$ | Cl | Cl | $OCF_3$ | H | Cl |
| NO | H | $(CH_2)_2Cl$ | Cl | Cl | $SCF_3$ | H | Cl |
| NO | H | $CF_3$ | Cl | Cl | Cl | H | Cl |
| NO | H | $CF_3$ | Cl | H | Cl | H | Cl |
| NO | $CH(CH_3)_2$ | $CH_3$ | Br | H | Br | H | Br |
| NO | H | $C_4F_9$ | Br | H | $CH_3$ | H | H |
| NO | H | $C_4H_9$ | Cl | H | $OCF_2CHF_2$ | H | H |

Tabelle 1 (Fortsetzung)

| $R^1$ | $R^2$ | $R^3$ | $R^4$ | $R^5$ | $R^6$ | $R^7$ | $R^8$ |
|---|---|---|---|---|---|---|---|
| NO | H | $CH_3$ | Cl | H | $SO_2CCl_2F$ | H | Cl |
| NO | H | $CH_3$ | Cl | H | $SO_2CClF_2$ | H | Cl |
| NO | $CH_3$ | $CH_2Cl$ | Br | H | $SCF_3$ | H | Br |
| NO | H | $C_4H_9$ | Br | H | $SCF_3$ | H | H |
| NO | H | $CH_3$ | $CF_3$ | F | Cl | H | Cl |
| NO | $CH_3$ | $CH_3$ | $CF_3$ | H | Cl | H | H |
| NO | H | $C_4H_9$ | $CF_3$ | H | Br | H | H |
| NO | H | $CH_3$ | Cl | F | CN | F | Cl |
| NO | H | $CH_3$ | F | F | CN | F | F |
| NO | H | $CH_3$ | F | Cl | F | Cl | F |
| NO | H | 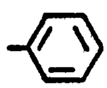 | Cl | Cl | $CF_3$ | H | Cl |
| NO | $CH_3$ | 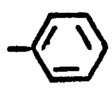 | Cl | H | $OCF_3$ | H | H |
| NO | $CH_3$ | 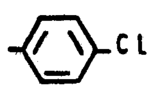 | Cl | H | $CF_3$ | H | Cl |
| NO | H | 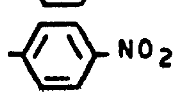 | Cl | H | $CF_3$ | H | Cl |
| NO | $CH_3$ | $CH_2Cl$ | Cl | H | $CF_3$ | H | H |
| NO | $C_3H_7$ | $CH_3$ | Cl | Cl | $CF_3$ | H | Cl |
| NO | $(CH_2)_2OH$ | $C_4H_9$ | Cl | H | $SO_2CF_3$ | H | Cl |

Tabelle 1 (Fortsetzung)

| $R^1$ | $R^2$ | $R^3$ | $R^4$ | $R^5$ | $R^6$ | $R^7$ | $R^8$ |
|---|---|---|---|---|---|---|---|
| NO | H | $CH_3$ | Cl | H | $SO_2CF_3$ | H | H |
| NO | H | $CH_3$ | Cl | H | $SOCF_3$ | H | H |
| NO | H | $CH_3$ | Cl | Cl | Cl | H | Cl |
| NO | H | $CF_3$ | Cl | H | $OCF_3$ | H | Cl |
| NO | $CH_3$ | $CF_3$ | Cl | Cl | Cl | H | H |
| NO | H | $C_4F_9$ | Cl | Cl | $CF_3$ | H | Cl |
| NO | $CH_3$ | $C_4F_9$ | Cl | H | $SCF_3$ | H | Cl |
| NO | H | $CH_3$ | $CF_3$ | H | $CF_3$ | H | H |
| NO | H | $CH_3$ | Cl | H | $NO_2$ | H | Cl |
| NO | H | $CH_3$ | $NO_2$ | H | $NO_2$ | H | H |
| NO | H | $CH_3$ | Cl | H | $CH_3$ | H | Cl |
| NO | H | $CF_3$ | Cl | H | $CH(CH_3)_2$ | H | Cl |
| NO | H | $CH_2Cl$ | Cl | H | $C(CH_3)_3$ | H | Cl |
| NO | H | $CH_3$ | Br | H | $OCF_3$ | H | Cl |
| $NO_2$ | H | $CH_3$ | Cl | H | $CF_3$ | H | Br |
| $NO_2$ | $CH_3$ | $CH_3$ | Cl | Cl | $CF_3$ | H | Cl |
| $NO_2$ | H | $CH_3$ | Cl | F | $CF_3$ | Cl | Cl |
| $NO_2$ | H | $CH_3$ | Cl | F | $CF_3$ | F | F |

Tabelle 1 (Fortsetzung)

| $R^1$ | $R^2$ | $R^3$ | $R^4$ | $R^5$ | $R^6$ | $R^7$ | $R^8$ |
|---|---|---|---|---|---|---|---|
| $NO_2$ | H | $CH_3$ | F | F | $CF_3$ | F | F |
| $NO_2$ | H | $CH_3$ | Cl | F | Cl | F | Cl |
| $NO_2$ | H | $C_2H_5$ | Cl | H | Br | H | Cl |
| $NO_2$ | H | $CH_3$ | F | F | F | F | F |
| $NO_2$ | $C_2H_5$ | $CH_2Cl$ | Cl | H | $CF_3$ | H | H |
| $NO_2$ | $(CH_2)_2OH$ | $CF_3$ | Cl | Cl | Cl | H | H |
| $NO_2$ | H | $CH_3$ | Cl | H | $OCF_3$ | H | Cl |
| $NO_2$ | H | $C_4H_9$ | Cl | H | $SCF_3$ | H | Cl |
| $NO_2$ | $CH_3$ | $CH_3$ | Cl | Cl | $OCF_3$ | H | Cl |
| $NO_2$ | H | $(CH_2)_2Cl$ | Cl | Cl | $SCF_3$ | H | Cl |
| $NO_2$ | H | $CF_3$ | Cl | Cl | Cl | H | Cl |
| $NO_2$ | H | $CF_3$ | Cl | H | Cl | H | Cl |
| $NO_2$ | $CH(CH_3)_2$ | $CH_3$ | Br | H | Br | H | Br |
| $NO_2$ | H | $C_4F_9$ | Br | H | $CH_3$ | H | H |
| $NO_2$ | H | $C_4H_9$ | Cl | H | $OCF_2CHF_2$ | H | H |
| $NO_2$ | H | $CH_3$ | Cl | H | $SO_2CCl_2F$ | H | Cl |
| $NO_2$ | H | $CH_3$ | Cl | H | $SO_2CClF_2$ | H | Cl |

Tabelle 1 (Fortsetzung)

| $R^1$ | $R^2$ | $R^3$ | $R^4$ | $R^5$ | $R^6$ | $R^7$ | $R^8$ |
|---|---|---|---|---|---|---|---|
| $NO_2$ | $CH_3$ | $CH_2Cl$ | Br | H | $SCF_3$ | H | Br |
| $NO_2$ | H | $C_4H_9$ | Br | H | $SCF_3$ | H | H |
| $NO_2$ | H | $CH_3$ | $CF_3$ | F | Cl | H | Cl |
| $NO_2$ | $CH_3$ | $CH_3$ | $CF_3$ | H | Cl | H | H |
| $NO_2$ | H | $C_4H_9$ | $CF_3$ | H | Br | H | H |
| $NO_2$ | H | $CH_3$ | Cl | F | CN | F | Cl |
| $NO_2$ | H | $CH_3$ | F | F | CN | F | F |
| $NO_2$ | H | $CH_3$ | F | Cl | F | Cl | F |
| $NO_2$ | H | 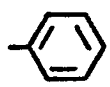 | Cl | Cl | $CF_3$ | H | Cl |
| $NO_2$ | $CH_3$ | 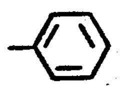 | Cl | H | $OCF_3$ | H | H |
| $NO_2$ | $CH_3$ | 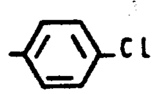 | Cl | H | $CF_3$ | H | Cl |
| $NO_2$ | H | 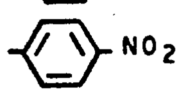 | Cl | H | $CF_3$ | H | Cl |
| $NO_2$ | $CH_3$ | $CH_2Cl$ | Cl | H | $CF_3$ | H | H |
| $NO_2$ | $C_3H_7$ | $CH_3$ | Cl | Cl | $CF_3$ | H | Cl |
| $NO_2$ | $(CH_2)_2OH$ | $C_4H_9$ | Cl | H | $SO_2CF_3$ | H | Cl |
| $NO_2$ | H | $CH_3$ | Cl | H | $SO_2CF_3$ | H | H |
| $NO_2$ | H | $CH_3$ | Cl | H | $SOCF_3$ | H | H |

0192951

Tabelle 1 (Fortsetzung)

| $R^1$ | $R^2$ | $R^3$ | $R^4$ | $R^5$ | $R^6$ | $R^7$ | $R^8$ |
|---|---|---|---|---|---|---|---|
| $NO_2$ | H | $CH_3$ | Cl | Cl | Cl | H | Cl |
| $NO_2$ | H | $CF_3$ | Cl | H | $OCF_3$ | H | Cl |
| $NO_2$ | $CH_3$ | $CF_3$ | Cl | Cl | Cl | H | H |
| $NO_2$ | H | $C_4F_9$ | Cl | Cl | $CF_3$ | H | Cl |
| $NO_2$ | $CH_3$ | $C_4F_9$ | Cl | H | $SCF_3$ | H | Cl |
| $NO_2$ | H | $CH_3$ | $CF_3$ | H | $CF_3$ | H | H |
| $NO_2$ | H | $CH_3$ | Cl | H | $NO_2$ | H | Cl |
| $NO_2$ | H | $CH_3$ | $NO_2$ | H | $NO_2$ | H | H |
| $NO_2$ | H | $CH_3$ | Cl | H | $CH_3$ | H | Cl |
| $NO_2$ | H | $CF_3$ | Cl | H | $CH(CH_3)_2$ | H | Cl |
| $NO_2$ | H | $CH_2Cl$ | Cl | H | $C(CH_3)_3$ | H | Cl |
| $NO_2$ | H | $CH_3$ | Br | H | $OCF_3$ | H | Cl |
| Cl | H | $CH_3$ | Cl | H | $CF_3$ | H | Br |
| Cl | $CH_3$ | $CH_3$ | Cl | Cl | $CF_3$ | H | Cl |
| Cl | H | $CH_3$ | Cl | F | $CF_3$ | Cl | Cl |
| Cl | H | $CH_3$ | Cl | F | $CF_3$ | F | F |
| Cl | H | $CH_3$ | F | F | $CF_3$ | F | F |

Tabelle 1 (Fortsetzung)

| $R^1$ | $R^2$ | $R^3$ | $R^4$ | $R^5$ | $R^6$ | $R^7$ | $R^8$ |
|---|---|---|---|---|---|---|---|
| Cl | H | $CH_3$ | Cl | F | Cl | F | Cl |
| Cl | H | $C_2H_5$ | Cl | H | Br | H | Cl |
| Cl | H | $CH_3$ | F | F | F | F | F |
| Cl | $C_2H_5$ | $CH_2Cl$ | Cl | H | $CF_3$ | H | H |
| Cl | $(CH_2)_2OH$ | $CF_3$ | Cl | Cl | Cl | H | H |
| Cl | H | $CH_3$ | Cl | H | $OCF_3$ | H | Cl |
| Cl | H | $C_4H_9$ | Cl | H | $SCF_3$ | H | Cl |
| Cl | $CH_3$ | $CH_3$ | Cl | Cl | $OCF_3$ | H | Cl |
| Cl | H | $(CH_2)_2Cl$ | Cl | Cl | $SCF_3$ | H | Cl |
| Cl | H | $CF_3$ | Cl | Cl | Cl | H | Cl |
| Cl | H | $CF_3$ | Cl | H | Cl | H | Cl |
| Cl | $CH(CH_3)_2$ | $CH_3$ | Br | H | Br | H | Br |
| Cl | H | $C_4F_9$ | Br | H | $CH_3$ | H | H |
| Cl | H | $C_4H_9$ | Cl | H | $OCF_2CHF_2$ | H | H |
| Cl | H | $CH_3$ | Cl | H | $SO_2CCl_2F$ | H | Cl |
| Cl | H | $CH_3$ | Cl | H | $SO_2CClF_2$ | H | Cl |

0192951

Tabelle 1 (Fortsetzung)

| $R^1$ | $R^2$ | $R^3$ | $R^4$ | $R^5$ | $R^6$ | $R^7$ | $R^8$ |
|---|---|---|---|---|---|---|---|
| Cl | $CH_3$ | $CH_2Cl$ | Br | H | $SCF_3$ | H | Br |
| Cl | H | $C_4H_9$ | Br | H | $SCF_3$ | H | H |
| Cl | H | $CH_3$ | $CF_3$ | F | Cl | H | Cl |
| Cl | $CH_3$ | $CH_3$ | $CF_3$ | H | Cl | H | H |
| Cl | H | $C_4H_9$ | $CF_3$ | H | Br | H | H |
| Cl | H | $CH_3$ | Cl | F | CN | F | Cl |
| Cl | H | $CH_3$ | F | F | CN | F | F |
| Cl | H | $CH_3$ | F | Cl | F | Cl | F |
| Cl | H | 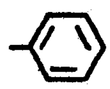 | Cl | Cl | $CF_3$ | H | Cl |
| Cl | $CH_3$ | 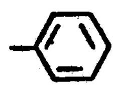 | Cl | H | $OCF_3$ | H | H |
| Cl | $CH_3$ | 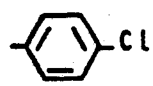 | Cl | H | $CF_3$ | H | Cl |
| Cl | H | 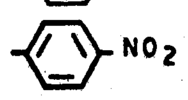 | Cl | H | $CF_3$ | H | Cl |
| Cl | $CH_3$ | $CH_2Cl$ | Cl | H | $CF_3$ | H | H |
| Cl | $C_3H_7$ | $CH_3$ | Cl | Cl | $CF_3$ | H | Cl |
| Cl | $(CH_2)_2OH$ | $C_4H_9$ | Cl | H | $SO_2CF_3$ | H | Cl |
| Cl | H | $CH_3$ | Cl | H | $SO_2CF_3$ | H | H |
| Cl | H | $CH_3$ | Cl | H | $SOCF_3$ | H | H |

Tabelle 1 (Fortsetzung)

| $R^1$ | $R^2$ | $R^3$ | $R^4$ | $R^5$ | $R^6$ | $R^7$ | $R^8$ |
|---|---|---|---|---|---|---|---|
| Cl | H | $CH_3$ | Cl | Cl | Cl | H | Cl |
| Cl | H | $CF_3$ | Cl | H | $OCF_3$ | H | Cl |
| Cl | $CH_3$ | $CF_3$ | Cl | Cl | Cl | H | H |
| Cl | H | $C_4F_9$ | Cl | Cl | $CF_3$ | H | Cl |
| Cl | $CH_3$ | $C_4F_9$ | Cl | H | $SCF_3$ | H | Cl |
| Cl | H | $CH_3$ | $CF_3$ | H | $CF_3$ | H | H |
| Cl | H | $CH_3$ | Cl | H | $NO_2$ | H | Cl |
| Cl | H | $CH_3$ | $NO_2$ | H | $NO_2$ | H | H |
| Cl | H | $CH_3$ | Cl | H | $CH_3$ | H | Cl |
| Cl | H | $CF_3$ | Cl | H | $CH(CH_3)_2$ | H | Cl |
| Cl | H | $CH_2Cl$ | Cl | H | $C(CH_3)_3$ | H | Cl |
| Cl | H | $CH_3$ | Br | H | $OCF_3$ | H | Cl |
| Br | H | $CH_3$ | Cl | H | $CF_3$ | H | Br |
| Br | $CH_3$ | $CH_3$ | Cl | Cl | $CF_3$ | H | Cl |
| Br | H | $CH_3$ | Cl | F | $CF_3$ | Cl | Cl |
| Br | H | $CH_3$ | Cl | F | $CF_3$ | F | F |
| Br | H | $CH_3$ | F | F | $CF_3$ | F | F |

Tabelle 1 (Fortsetzung)

| $R^1$ | $R^2$ | $R^3$ | $R^4$ | $R^5$ | $R^6$ | $R^7$ | $R^8$ |
|---|---|---|---|---|---|---|---|
| Br | H | $CH_3$ | Cl | F | Cl | F | Cl |
| Br | H | $C_2H_5$ | Cl | H | Br | H | Cl |
| Br | H | $CH_3$ | F | F | F | F | F |
| Br | $C_2H_5$ | $CH_2Cl$ | Cl | H | $CF_3$ | H | H |
| Br | $(CH_2)_2OH$ | $CF_3$ | Cl | Cl | Cl | H | H |
| Br | H | $CH_3$ | Cl | H | $OCF_3$ | H | Cl |
| Br | H | $C_4H_9$ | Cl | H | $SCF_3$ | H | Cl |
| Br | $CH_3$ | $CH_3$ | Cl | Cl | $OCF_3$ | H | Cl |
| Br | H | $(CH_2)_2Cl$ | Cl | Cl | $SCF_3$ | H | Cl |
| Br | H | $CF_3$ | Cl | Cl | Cl | H | Cl |
| Br | H | $CF_3$ | Cl | H | Cl | H | Cl |
| Br | $CH(CH_3)_2$ | $CH_3$ | Br | H | Br | H | Br |
| Br | H | $C_4F_9$ | Br | H | $CH_3$ | H | H |
| Br | H | $C_4H_9$ | Cl | H | $OCF_2CHF_2$ | H | H |
| Br | H | $CH_3$ | Cl | H | $SO_2CCl_2F$ | H | Cl |
| Br | H | $CH_3$ | Cl | H | $SO_2CClF_2$ | H | Cl |

Tabelle 1 (Fortsetzung)

| $R^1$ | $R^2$ | $R^3$ | $R^4$ | $R^5$ | $R^6$ | $R^7$ | $R^8$ |
|---|---|---|---|---|---|---|---|
| Br | $CH_3$ | $CH_2Cl$ | Br | H | $SCF_3$ | H | Br |
| Br | H | $C_4H_9$ | Br | H | $SCF_3$ | H | H |
| Br | H | $CH_3$ | $CF_3$ | F | Cl | H | Cl |
| Br | $CH_3$ | $CH_3$ | $CF_3$ | H | Cl | H | H |
| Br | H | $C_4H_9$ | $CF_3$ | H | Br | H | H |
| Br | H | $CH_3$ | Cl | F | CN | F | Cl |
| Br | H | $CH_3$ | F | F | CN | F | F |
| Br | H | $CH_3$ | F | Cl | F | Cl | F |
| Br | H | 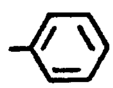 | Cl | Cl | $CF_3$ | H | Cl |
| Br | $CH_3$ | 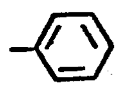 | Cl | H | $OCF_3$ | H | H |
| Br | $CH_3$ | 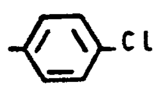 | Cl | H | $CF_3$ | H | Cl |
| Br | H | 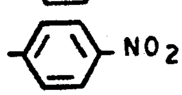 | Cl | H | $CF_3$ | H | Cl |
| Br | $CH_3$ | $CH_2Cl$ | Cl | H | $CF_3$ | H | H |
| Br | $C_3H_7$ | $CH_3$ | Cl | Cl | $CF_3$ | H | Cl |
| Br | $(CH_2)_2OH$ | $C_4H_9$ | Cl | H | $SO_2CF_3$ | H | Cl |
| Br | H | $CH_3$ | Cl | H | $SO_2CF_3$ | H | H |
| Br | H | $CH_3$ | Cl | H | $SOCF_3$ | H | H |

Tabelle 1 (Fortsetzung)

| $R^1$ | $R^2$ | $R^3$ | $R^4$ | $R^5$ | $R^6$ | $R^7$ | $R^8$ |
|---|---|---|---|---|---|---|---|
| Br | H | $CH_3$ | Cl | Cl | Cl | H | Cl |
| Br | H | $CF_3$ | Cl | H | $OCF_3$ | H | Cl |
| Br | $CH_3$ | $CF_3$ | Cl | Cl | Cl | H | H |
| Br | H | $C_4F_9$ | Cl | Cl | $CF_3$ | H | Cl |
| Br | $CH_3$ | $C_4F_9$ | Cl | H | $SCF_3$ | H | Cl |
| Br | H | $CH_3$ | $CF_3$ | H | $CF_3$ | H | H |
| Br | H | $CH_3$ | Cl | H | $NO_2$ | H | Cl |
| Br | H | $CH_3$ | $NO_2$ | H | $NO_2$ | H | H |
| Br | H | $CH_3$ | Cl | H | $CH_3$ | H | Cl |
| Br | H | $CF_3$ | Cl | H | $CH(CH_3)_2$ | H | Cl |
| Br | H | $CH_2Cl$ | Cl | H | $C(CH_3)_3$ | H | Cl |
| Br | H | $CH_3$ | Br | H | $OCF_3$ | H | Cl |
| I | H | $CH_3$ | Cl | H | $CF_3$ | H | Br |
| I | $CH_3$ | $CH_3$ | Cl | Cl | $CF_3$ | H | Cl |
| I | H | $CH_3$ | Cl | F | $CF_3$ | Cl | Cl |
| I | H | $CH_3$ | Cl | F | $CF_3$ | F | F |
| I | H | $CH_3$ | F | F | $CF_3$ | F | F |

Tabelle 1 (Fortsetzung)

| $R^1$ | $R^2$ | $R^3$ | $R^4$ | $R^5$ | $R^6$ | $R^7$ | $R^8$ |
|---|---|---|---|---|---|---|---|
| I | H | $CH_3$ | Cl | F | Cl | F | Cl |
| I | H | $C_2H_5$ | Cl | H | Br | H | Cl |
| I | H | $CH_3$ | F | F | F | F | F |
| I | $C_2H_5$ | $CH_2Cl$ | Cl | H | $CF_3$ | H | H |
| I | $(CH_2)_2OH$ | $CF_3$ | Cl | Cl | Cl | H | H |
| I | H | $CH_3$ | Cl | H | $OCF_3$ | H | Cl |
| I | H | $C_4H_9$ | Cl | H | $SCF_3$ | H | Cl |
| I | $CH_3$ | $CH_3$ | Cl | Cl | $OCF_3$ | H | Cl |
| I | H | $(CH_2)_2Cl$ | Cl | Cl | $SCF_3$ | H | Cl |
| I | H | $CF_3$ | Cl | Cl | Cl | H | Cl |
| I | H | $CF_3$ | Cl | H | Cl | H | Cl |
| I | $CH(CH_3)_2$ | $CH_3$ | Br | H | Br | H | Br |
| I | H | $C_4F_9$ | Br | H | $CH_3$ | H | H |
| I | H | $C_4H_9$ | Cl | H | $OCF_2CHF_2$ | H | H |
| I | H | $CH_3$ | Cl | H | $SO_2CCl_2F$ | H | Cl |
| I | H | $CH_3$ | Cl | H | $SO_2CClF_2$ | H | Cl |

Tabelle 1 (Fortsetzung)

| $R^1$ | $R^2$ | $R^3$ | $R^4$ | $R^5$ | $R^6$ | $R^7$ | $R^8$ |
|---|---|---|---|---|---|---|---|
| I | $CH_3$ | $CH_2Cl$ | Br | H | $SCF_3$ | H | Br |
| I | H | $C_4H_9$ | Br | H | $SCF_3$ | H | H |
| I | H | $CH_3$ | $CF_3$ | F | Cl | H | Cl |
| I | $CH_3$ | $CH_3$ | $CF_3$ | H | Cl | H | H |
| I | H | $C_4H_9$ | $CF_3$ | H | Br | H | H |
| I | H | $CH_3$ | Cl | F | CN | F | Cl |
| I | H | $CH_3$ | F | F | CN | F | F |
| I | H | $CH_3$ | F | Cl | F | Cl | F |
| I | H | 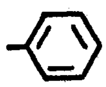 | Cl | Cl | $CF_3$ | H | Cl |
| I | $CH_3$ | 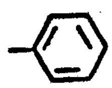 | Cl | H | $OCF_3$ | H | H |
| I | $CH_3$ | 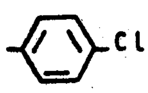 | Cl | H | $CF_3$ | H | Cl |
| I | H | 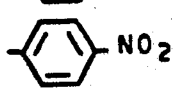 | Cl | H | $CF_3$ | H | Cl |
| I | $CH_3$ | $CH_2Cl$ | Cl | H | $CF_3$ | H | H |
| I | $C_3H_7$ | $CH_3$ | Cl | Cl | $CF_3$ | H | Cl |
| I | $(CH_2)_2OH$ | $C_4H_9$ | Cl | H | $SO_2CF_3$ | H | Cl |

0192951

Tabelle 1 (Fortsetzung)

| $R^1$ | $R^2$ | $R^3$ | $R^4$ | $R^5$ | $R^6$ | $R^7$ | $R^8$ |
|---|---|---|---|---|---|---|---|
| I | H | $CH_3$ | Cl | H | $SO_2CF_3$ | H | H |
| I | H | $CH_3$ | Cl | H | $SOCF_3$ | H | H |
| I | H | $CH_3$ | Cl | Cl | Cl | H | Cl |
| I | H | $CF_3$ | Cl | H | $OCF_3$ | H | Cl |
| I | $CH_3$ | $CF_3$ | Cl | Cl | Cl | H | H |
| I | H | $C_4F_9$ | Cl | Cl | $CF_3$ | H | Cl |
| I | $CH_3$ | $C_4F_9$ | Cl | H | $SCF_3$ | H | Cl |
| I | H | $CH_3$ | $CF_3$ | H | $CF_3$ | H | H |
| I | H | $CH_3$ | Cl | H | $NO_2$ | H | Cl |
| I | H | $CH_3$ | $NO_2$ | H | $NO_2$ | H | H |
| I | H | $CH_3$ | Cl | H | $CH_3$ | H | Cl |
| I | H | $CF_3$ | Cl | H | $CH(CH_3)_2$ | H | Cl |
| I | H | $CH_2Cl$ | Cl | H | $C(CH_3)_3$ | H | Cl |
| I | H | $CH_3$ | Br | H | $OCF_3$ | H | Cl |

Verwendet man beispielsweise 5-Amino-1-(2,6-dichlor-4-trifluormethyl)-phenyl-pyrazol und Methansulfonsäurechlorid als Ausgangsstoffe, so läßt sich der Reaktionsablauf des erfindungsgemäßen Verfahrens (a) durch das folgende Formelschema darstellen:

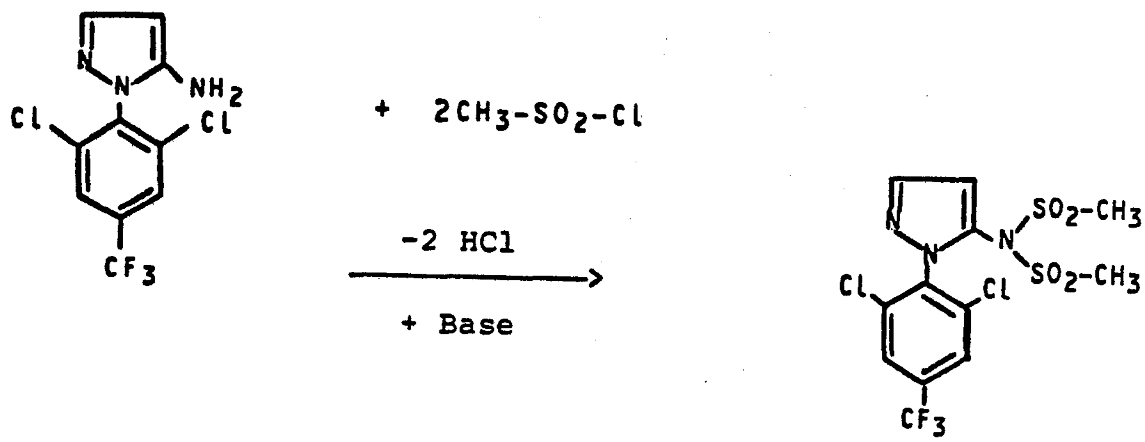


Verwendet man beispielsweise 5-/N,N-Bis-(methansulfonyl)-amino/-1-(2,6-dichlor-4-trifluormethyl)-phenyl-pyrazol und Ammoniak als Ausgangsstoffe, so läßt sich der Reaktionsablauf des erfindungsgemäßen Verfahrens (b) durch das folgende Formelschema darstellen:

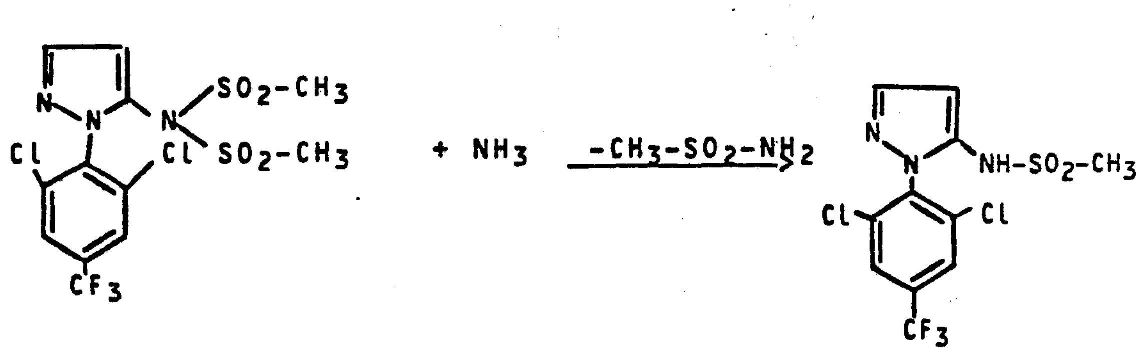

Verwendet man beispielsweise 5-Methansulfonamido-1-(2,6-dichlor-4-trifluormethyl)-phenyl-pyrazol und Salpetersäure als Ausgangsstoffe, so läßt sich der Reaktionsablauf des erfindungsgemäßen Verfahrens (c) durch das folgende Formelschema darstellen:

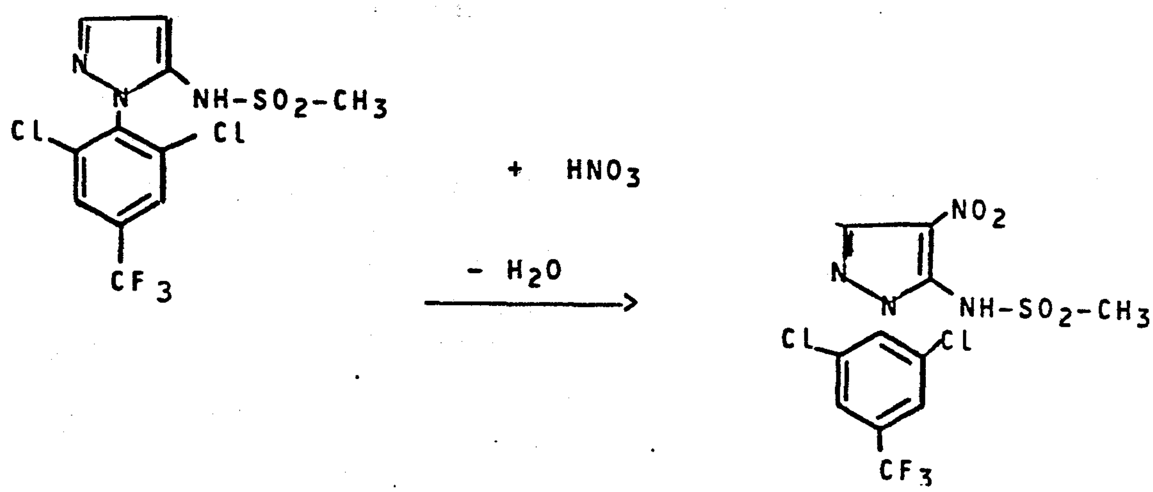


Verwendet man beispielsweise 4-Nitro-5-methansulfonamido-1-(2,6-dichlor-4-trifluormethyl)phenyl-pyrazol und Methyliodid als Ausgangsstoffe, so läßt sich der Reaktionsablauf des erfindungsgemäßen Verfahrens (d-α) durch das folgende Formelschema darstellen:

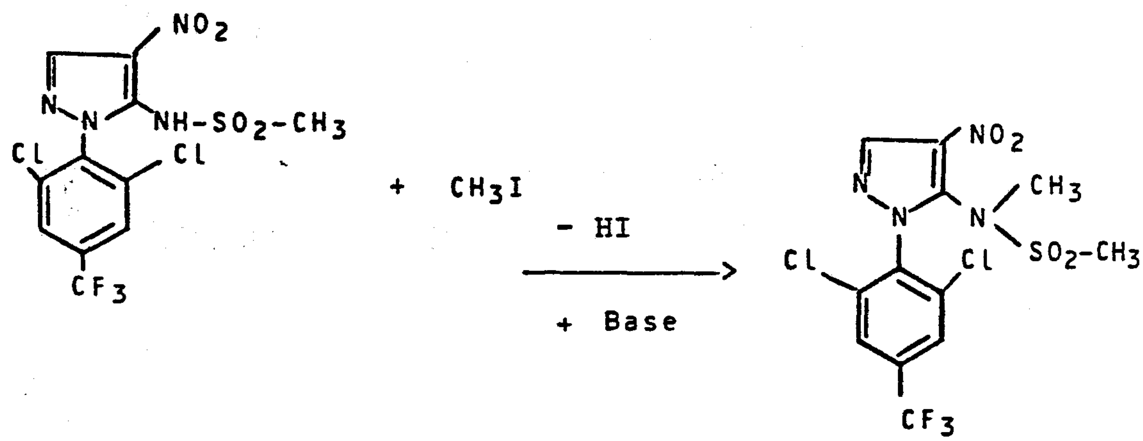

Verwendet man beispielsweise 4-Chlor-5-methan-sulfonamido-1-(2-chlor-4-trifluormethyl)-phenyl-pyrazol und Isopropylamin als Ausgangsstoffe, so läßt sich der Reaktionsablauf des erfindungsgemäßen Verfahrens (d-ß) durch das folgende Formelschema darstellen:

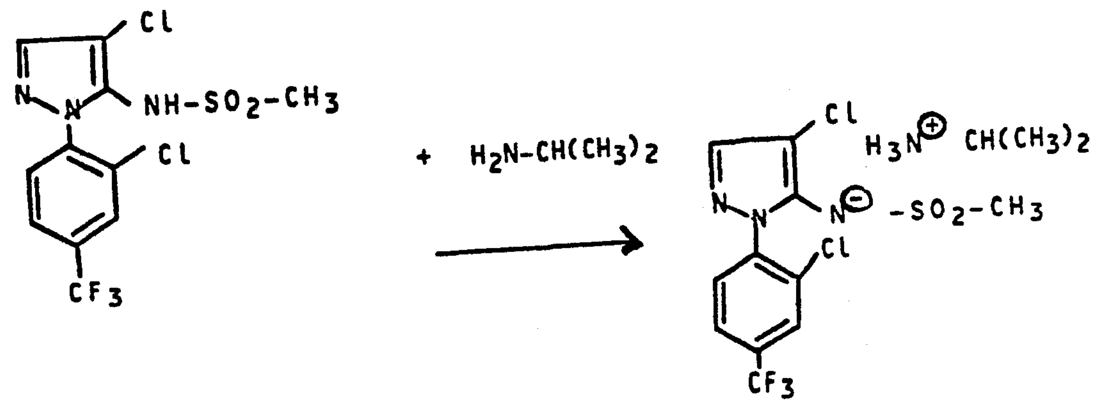

Die zur Durchführung des erfindungsgemäßen Verfahrens (a) als Ausgangsstoffe benötigten 5-Aminopyrazole sind durch die Formel (II) allgemein definiert. In dieser Formel (II) stehen $R^4$, $R^5$, $R^6$, $R^7$ und $R^8$ vorzugsweise für diejenigen Substituenten, die bereits im Zusammenhang mit der Beschreibung der erfindungsgemäßen Stoffe der Formel (I) als bevorzugt für diese Reste genannte wurden.

Die 5-Aminopyrazole der Formel (II) sind teilweise bekannt (vgl. z.B. J. Org. Chemistry 36, 2972-2974 (1971); J. Heterocycl. Chem. 7, 345-349 (1970); C.A. 62 13 137c).

Die noch nicht bekannten substituierten 5-Aminopyrazole der Formel (II) sind jedoch Gegenstand einer eigenen vorgängigen Patentanmeldung (DE-P 3 402 308 vom 24.01.84).

Man erhält sie beispielsweise, wenn man Phenylhydrazine der Formel (VII),

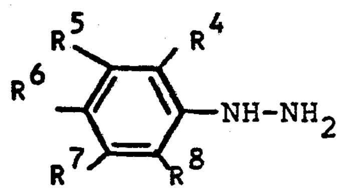

(VII)

in welcher

$R^4$, $R^5$, $R^6$, $R^7$ und $R^8$ die oben angegebene Bedeutung haben,

mit 2-Halogen-acrylnitrilen der Formel (VIIIa),

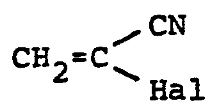

(VIIIa)

in welcher

Hal für Halogen, insbesondere für Chlor oder Brom steht,

oder

mit 2,3-Dihalogenpropionitrilen der Formel (VIIIb)

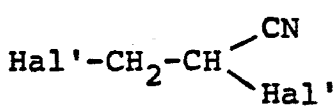

(VIIIb)

in welcher

Hal' für Halogen, insbesondere für Chlor oder Brom steht,

entweder zunächst in einer 1. Stufe, gegebenenfalls in Gegenwart eines Verdünnungsmittels, wie beispielsweise Methanol, sowie gegebenenfalls in Gegenwart eines Reaktionshilfsmittels, wie beispielsweise Schwefelsäure, bei Temperaturen zwischen -30°C und +50°C umsetzt zu den Phenylhydrazin-Derivaten der Formel (IX),

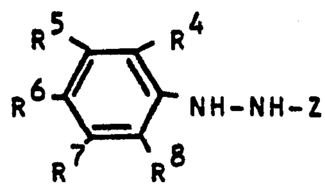

(IX)

in welcher

$R^4$, $R^5$, $R^6$, $R^7$ und $R^8$ die oben angegebene Bedeutung haben und

Z für einen der Reste 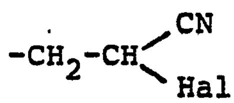 oder

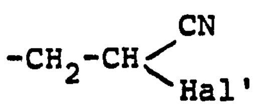 steht, wobei Hal und Hal' für gleiche oder unterschiedliche Halogenatome stehen,

und diese in einer 2. Stufe, gegebenenfalls in Gegenwart eines Verdünnungsmittels, wie beispielsweise Methanol, und gegebenenfalls in Gegenwart eines Säurebindemittels, wie beispielsweise Natriumcarbonat, bei Temperaturen zwischen 50°C und 150°C cyclisiert, oder direkt in einem Reaktionsschritt ohne Isolierung der Zwischenstufe der Formel (IX), gegebenenfalls in Gegenwart eines Verdünnungsmittels, wie beispielsweise Ethanol, bei Temperaturen zwischen 50°C und 150°C direkt cyclisiert zu den 5-Amino-pyrazolen der

Formel (II),

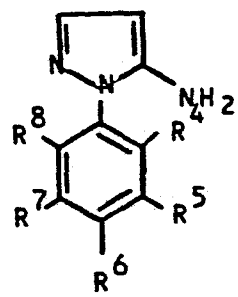

(II)

in welcher

$R^4$, $R^5$, $R^6$, $R^7$ und $R^8$ die oben angegebene Bedeutung haben.

Die Phenylhydrazine der Formel (VII) sind größenteils bekannt oder können nach bekannten Verfahren in einfacher, analoger Weise hergestellt werden (vgl. z.B. Houben-Weyl "Methoden der organischen Chemie" Band X,2, Thieme Verlag Stuttgart 1967), indem man beispielsweise die bekannten Aniline der Formel (X),

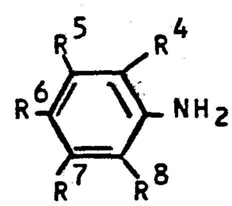

(X)

in welcher

$R^4$, $R^5$, $R^6$, $R^7$ und $R^8$ die oben angegebene Bedeutung haben,

mit Natriumnitrit in Gegenwart einer Säure, wie beispielsweise Schwefelsäure, und dann mit Zinn-(II)-chlorid, ebenfalls in Gegenwart einer Säure, wie beispielsweise Salzsäure, bei Temperaturen zwischen -20°C und +80°C umsetzt.

Die 2-Halogen-acrylnitrile der Formel (VIIIa) und die 2,3-Dihalogen-propionitrile der Formel (VIIIb) sind ebenfalls bekannt (vgl. z.B. J. Prakt. Chemie 321, 93 (1979); J. Heterocyclic Chem. 19, 1265 (1982); J. Heterocyclic Chem. 19, 1267 (1982)).

Die zur Durchführung des erfindungsgemäßen Verfahrens (a) weiterhin als Ausgangsstoffe benötigten Sulfonylchloride sind durch die Formel (III) allgemein definiert. Bevorzugt sind Verbindungen der Formel (III), bei welchen $R^3$ vorzugsweise für diejenigen Reste steht, die bereits bei der Beschreibung der erfindungsgemäßen Stoffe der Formel (I) als bevorzugt für diesen Substituenten genannt wurden.

Die Sulfonylchloride der Formel (III) sind allgemein bekannte Verbindungen der organischen Chemie.

Die zur Durchführung des erfindungsgemäßen Verfahrens (b) als Ausgangsstoffe benötigten Bisulfonylamine sind durch die Formel (Ia) allgemein definiert. In dieser Formel (Ia) stehen $R^3$, $R^4$, $R^5$, $R^6$, $R^7$ und $R^8$ vorzugsweise für diejenigen Substituenten, die bereits bei der Beschreibung der erfindungsgemäßen Stoffe der Formel (I) als bevorzugt für diese Reste genannt wurden.

Die Bisulfonylamine sind erfindungsgemäße Stoffe und erhältlich mit der Hilfe des erfindungsgemäßen Verfahrens (a).

Die zur Durchführung des erfindungsgemäßen Verfahrens (c) als Ausgangsstoffe benötigten 5-Sulfonamido-pyrazole sind durch die Formel (Ib) allgemein definiert. In dieser Formel (Ib) stehen $R^3$, $R^4$, $R^5$, $R^6$, $R^7$ und $R^8$ vorzugsweise für diejenigen Substituenten, die bereits im Zusammenhang mit der Beschreibung der erfindungsgemäßen Stoffe der Formel (I) als bevorzugt für diese Reste genannte wurden. $R^{2'}$ steht vorzugsweise für Wasserstoff oder für einen Rest $R^3$-$SO_2$-.

Die 5-Sulfonamido-pyrazole der Formel (Ib) sind erfindungsgemäße Stoffe und erhältlich mit Hilfe der erfindungsgemäßen Verfahren (a) oder (b).

Die zur Durchführung des erfindungsgemäßen Verfahrens (c) weiterhin als Ausgangsstoffe benötigten elektrophilen Agenzien sind durch die Formel (IV) allgemein definiert. In dieser Formel (IV) steht $R^{1'}$ vorzugsweise für Chlor, Brom, Iod, Nitroso oder Nitro. E steht vorzugsweise für Halogen, insbesondere Chlor oder Brom, für Hydroxy, für Alkyl, für Alkyl- oder Arylsulfonyloxy, für Alkanoyloxy oder Aroyloxy. Weiterhin verwendbare elektrophile Reagenzien sind Sulfurylchlorid, Phosphorpentachlorid, Nitriersäure und andere üblicherweise zu elektrophilen Substitutionen verwendbaren Stoffe. Die elektrophilen Agenzien der Formel (IV) ebenso wie die weiteren üblichen elektrophilen Reagenzien sind allgemein bekannte Verbindungen.

Die zur Durchführung des erfindungsgemäßen Verfahrens (d) als Ausgangsstoffe benötigten 5-Sulfonamido-pyrazole sind durch die Formel (Ic) allgemein definiert. In

dieser Formel (Ic) stehen $R^1$, $R^3$, $R^4$, $R^5$, $R^6$, $R^7$ und $R^8$ vorzugsweise für diejenigen Substituenten, die bereits im Zusammenhang mit der Beschreibung der erfindungsgemäßen Stoffe der Formel (I) als bevorzugt für diese Reste genannt wurden.

Die 5-Sulfonamido-pyrazole der Formel (Ic) sind erfindungsgemäße Verbindungen und erhältlich mit Hilfe der erfindungsgemäßen Verfahren (a), (b) oder (c).

Die zur Durchführung des erfindungsgemäßen Verfahrens (d-$\alpha$) weiterhin als Ausgangsverbindungen benötigten Alkylierungsmittel sind durch die Formel (V) allgemein definiert. In dieser Formel (V) steht $R^{2''}$ vorzugsweise für geradkettiges oder verzweigtes Alkyl mit 1 bis 8 Kohlenstoffatomen, insbesondere für Methyl, Ethyl, n- oder i-Propyl, sowie n-, i-, s- oder t-Butyl. E' steht vorzugsweise für Chlor, Brom oder Iod, p-Toluolsulfonyloxy oder Alkoxysulfonyloxy, insbesondere für Chlor, Brom, Iod oder Methoxysulfonyloxy bzw. Ethoxysulfonyloxy. Die Alkylierungsmittel der Formel (V) sind allgemein bekannte Verbindungen der organischen Chemie.

Die zur Durchführung des erfindungsgemäßen Verfahrens (d-ß) weiterhin als Ausgangsstoffe benötigten Salzbildner sind durch die Formel (VI) allgemein definiert. Vorzugsweise verwendet man Alkali-, Erdalkali-, Ammonium- oder Übergangsmetallhydroxide, -oxide, -carbonate, -hydrogencarbonate oder leicht lösliche -chloride, -sulfate, -phosphate oder -nitrate, wie beispielsweise

Natrium-, Kalium-, Calciumhydroxid, -carbonat oder -hydrogencarbonat, Calciumchlorid, Bariumchlorid, Kupfersulfat, Nickelchlorid oder Cobaltnitrat oder Alkylamine, wie Triethylamin, Isopropylamin, Diisopropylamin, Butylamin.

Als Verdünnungsmittel zur Durchführung des erfindungsgemäßen Verfahrens (a) kommen inerte organische Lösungsmittel infrage.

Vorzugsweise verwendet man aliphatische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Nitrile, wie Acetonitril oder Propionitril, Amide, wie Dimethylformamid, Diethylformamid, Dimethylacetamid, N-Methylpyrrolidon oder Hexamethylphosphorsäuretriamid.

Als Säurebindemittel zur Durchführung des Herstellungsverfahrens (a) kommen alle üblicherweise verwendbaren anorganischen und organischen Basen infrage. Vorzugsweise verwendet man Alkalimetallhydride-, -hydroxide, -amide, -carbonate oder -hydrogencarbonate, wie beispielsweise Natriumhydrid, Natriumamid, Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des Herstellungsverfahrens (a) in einem größeren Bereich variiert werden. Im allgemeinen arbeitet man zwischen -20°C und 150°C, vorzugsweise zwischen 0°C und +100°C.

Zur Durchführung des Herstellungsverfahrens (a) setzt man pro Mol 5-Amino-pyrazol der Formel (II) im allgemeinen 1,0 bis 20 Mol, vorzugsweise 1,0 bis 15 Mol an Sulfonylchlorid der Formel (III) und gegebenenfalls 1,0 bis 3,0 Mol, vorzugsweise 1,0 bis 2,0 Mol an Säurebindemittel ein. Die Reaktionsdurchführung, Aufarbeitung und Isolierung der Reaktionsprodukte der Formel (I) erfolgt in allgemein üblicher Art und Weise.

Als Verdünnungsmittel zur Durchführung des erfindungsgemäßen Verfahrens (b) kommen polare organische Lösungsmittel oder deren Gemische mit Wasser infrage. Vorzugsweise verwendet man Alkohole wie Methanol, Ethanol oder Propanol oder deren Gemische mit Wasser.

Als basische Reaktionsteilnehmer bei der Durchführung des erfindungsgemäßen Verfahrens (b) kommen alle üblichen anorganischen oder organischen Basen infrage. Vorzugsweise verwendet man Amine oder Ammoniaklösungen oder Alkalimetallcarbonate bzw. -hydrogencarbonate, wie Natrium- oder Kaliumcarbonat oder Natriumhydrogencarbonat.

Die Reaktionstemperaturen können bei der Durchführung des erfindungsgemäßen Verfahrens (b) in einem größeren Bereich variiert werden. Im allgemeinen arbeitet man zwischen 0°C und 80°C, vorzugsweise zwischen 20°C und 40°C.

Zur Durchführung des erfindungsgemäßen Verfahrens (b) setzt man pro Mol Bis-sulfonylamin der Formel (Ia) im allgemeinen 1,0 bis 30,0 Mol, vorzugsweise 1,0 bis 15,0 Mol, an Base ein.

Die Reaktionsmischung wird in einem geeigneten Verdünnungsmittel so lange gerührt (30 Minuten bis 20 Stunden) bis bei chromatographischer Kontrolle kein Ausgangsprodukt mehr nachweisbar ist. Die Aufarbeitung der Reaktionsprodukte der Formel (I) erfolgt nach üblichen Methoden.

Als Verdünnungsmittel zur Durchführung des Herstellungsverfahrens (c) kommen alle üblicherweise für derartige elektrophile Substitutionen verwendbaren Lösungsmittel infrage. Vorzugsweise verwendet man die als Reagenzien

infrage kommenden Säuren oder Gemische, wie beispielsweise Schwefelsäure, Salpetersäure, Sulfurylchlorid oder Nitriersäure gleichzeitig als Verdünnungsmittel. Es kommen gegebenenfalls auch inerte organische Lösungsmittel, wie beispielsweise Eisessig, Ethanol oder chlorierte Kohlenwasserstoffe, wie Methylenchlorid, Chloroform oder Tetrachlorkohlenstoff, als Verdünnungsmittel infrage.

Als Katalysatoren oder Reaktionshilfsmittel zur Durchführung des Herstellungsverfahrens (c) kommen ebenfalls die für derartige Reaktionen üblichen Katalysatoren infrage; vorzugsweise Salzsäure, Schwefelsäure, Eisen-II-chlorid oder andere Lewis-Säuren oder Acetanhydrid.

Die Reaktionstemperaturen können bei der Durchführung des Herstellungsverfahrens (c) in einem größeren Bereich variiert werden. Im allgemeinen arbeitet man zwischen -50°C und +200°C, vorzugsweise zwischen -20°C und +150°C.

Zur Durchführung des Herstellungsverfahrens (c) setzt man pro Mol 5-Amino-pyrazol der Formel (Ib) im allgemeinen 1,0 bis 10,0 Mol, vorzugsweise 1,0 bis 5,0 Mol, an elektrophilem Agens der Formel (IV) und gegebenenfalls 0,1 bis 10 Mol an Katalysator oder Reaktionshilfsmittel ein. Die Reaktionsführung, Aufarbeitung und Isolierung der Reaktionsprodukte der Formel (I) erfolgt in allgemein üblicher Art und Weise.

Als Verdünnungsmittel zur Durchführung des Herstellungsverfahrens (d-α) kommen ebenfalls inerte organische Lösungsmittel infrage. Vorzugsweise verwendet man die bei dem Herstellungverfahren (a) genannten Lösungsmittel.

Als Säurebindemittel oder Katalysator zur Durchführung des Herstellungsverfahrens (d-α) kommen alle üblicherweise verwendbaren organischen oder anorganischen Basen infrage. Vorzugsweise verwendet man die bei Verfahren (a) genannten Basen.

Die Reaktionstemperaturen können bei dem Herstellungsverfahren (d-α) ebenfalls in einem größeren Bereich variiert werden. Im allgemeinen arbeitet man zwischen -20°C und +120°C, vorzugsweise zwischen 0°C und +90°C.

Zur Durchführung des Herstellungsverfahrens (d-α) setzt man pro Mol 5-Sulfonamidopyrazol der Formel (Ic) im allgemeinen 1,0 bis 5,0 Mol, vorzugsweise 1,0 bis 3,0 Mol, an Alkylierungsmittel der Formel (V) und gegebenenfalls 1,0 bis 5,0 Mol, vorzugsweise 1,0 bis 3,0 Mol, an Säurebindemittel ein. Die Reaktionsführung, Aufarbeitung und Isolierung der Reaktionsprodukte der Formel (I) erfolgt in üblicher Art und Weise.

Als Verdünnungsmittel zur Durchführung des Herstellungsverfahrens (d-ß) kommen polare organische Lösungsmittel, Wasser oder wäßrige Gemische infrage. Vorzugsweise verwendet man Alkohole, wie beispielsweise Methanol, Ethanol oder Propanol, deren wäßrige Gemische oder reines Wasser.

Die Reaktionstemperaturen können bei der Durchführung des Herstellungsverfahrens (d-ß) ebenfalls in einem größeren Bereich variiert werden. Im allgemeinen arbeitet man zwischen 0°C und +80°C, vorzugsweise zwischen +20°C und +40°C.

Zur Durchführung des erfindungsgemäßen Verfahrens (d-ß) setzt man pro Mol 5-Sulfonamidopyrazol der Formel (Ic) im allgemeinen 1,0 bis 10 Mol, vorzugsweise 1,0 bis 5,0 Mol an Salzbildner der Formel (VI) oder Amin ein.

Zur Herstellung der Natrium-, Kalium- oder Ammoniumsalze setzt man eine Verbindung der Formel (Ic) in wäßriger Lösung oder einem organischen Lösungsmittel, wie Aceton, Methanol, Ethanol oder Dimethylformamid, mit Natrium-, Kalium- oder Ammoniumhydroxid oder einem Amin um und isoliert die Salze durch Abfiltrieren oder durch Eindampfen der Lösung und reinigt sie gegebenenfalls durch Umkristallisieren.

Die Calcium-, Barium-, Magnesium-, Mangan-, Kupfer-, Nickel-, Zinn-, Eisen- und Cobaltsalze werden hergestellt aus den Natriumsalzen durch Behandeln mit einem entsprechenden anorganischen Metallsalz, z.B. Calciumchlorid, Bariumchlorid, Kupfersulfat, Nickelchlorid oder Cobaltnitrit. Die Calciumsalze können auch hergestellt werden durch Behandeln einer Verbindung der Formel (Ic) mit Calciumhydroxid.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung mono- und dikotyler Unkräuter in monokotylen Kulturen wie beispielsweise Weizen einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

0192951

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit N,N-Dimethyl-N'-(3-trifluormethylphenyl)-harnstoff, N,N-Dimethyl-N'-(3-chlor-4-methylphenyl)-harnstoff; N,N-Dimethyl-N'-(4-isopropylphenyl)-harnstoff; 2,4-Dichlorphenoxyessigsäure; 2,4-Dichlorphenoxypropionsäure; (2-Methyl-4-chlorphenoxy)-essigsäure; (4-Chlor-2-methyl-phenoxy)-propionsäure; 2-/4-(3,5-Dichlorpyrid-2-yloxy)-phenoxy7-propionsäure-(2-benzyloxy-ethylester), -(trimethylsilylmethylester) oder -(2,2-diethoxyethylester); Methyl-5-(2,4-dichlorphenoxy)-2-nitrobenzoat; 3,5-Diiod-4-hydroxybenzonitril; 3-Isopropyl-2,1,3-benzothiadiazin-4-on-2,2-dioxid; 2-Chlor-N- </(4-methoxy-6-methyl-1,3,5-triazin-2-yl)-amino7-carbonyl >-benzolsulfonamid, 4-Ethylamino-2-t-butylamino-6-methylthio-s-triazin; N-Methyl-2-(benzthiazol-2-yloxy)-acetamid; N-(1-Ethylpropyl)-3,4-dimethyl-2,6-dinitroanilin; α-Chlor-1',6'-diethyl-N-(2-propoxyethyl)-acetanilid oder N,N-Diisopropyl-(2,3,3-trichlorallyl)-thiocarbamat, sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

0192951

## Herstellungsbeispiele:

### Beispiel 1

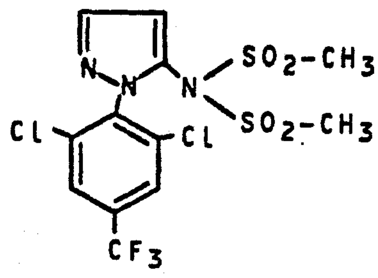

nach Verfahren (a):

5,9 g (0,02 Mol) 5-Amino-1-(2,6-dichlor-4-trifluormethylphenyl)-pyrazol werden in 40 ml Methylenchlorid gelöst und tropfenweise mit 4 ml (≙ 3,9 g ≙ 0,047 Mol) Methansulfonylchlorid versetzt. Es wird 8 Stunden unter Rückfluß erhitzt, abgekühlt und nacheinander mit Wasser, verdünnter Salzsäure und gesättigter Bicarbonat-Lösung gewaschen. Die organische Phase wird über Magnesiumsulfat getrocknet und im Vakuum eingedampft.

Man erhält 7,6 g Rohprodukt, das aus mono- und bimesyliertem Aminopyrazol besteht. Das gesamte Rohprodukt wird in ca. 20 ml Ethanol verrührt. Der unlösliche Rückstand wird filtriert, gewaschen und getrocknet.

Man erhält 1,3 g (14 % der Theorie) 5-Bis-(methansulfon)-imido-1-(2,6-dichlor-4-trifluormethylphenyl)pyrazol vom Schmelzpunkt 196-198°C.

Beispiel 2

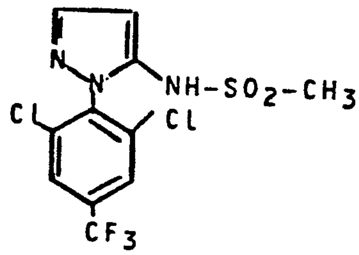

nach Verfahren (a):

Das ethanollösliche Filtrat aus Beispiel 1 wird mit etwas Aktivkohle versetzt, filtriert und eingedampft.

Man erhält ein bräunliches Öl, das langsam auskristallisiert. Ausbeute: 5,0 g (67 % der Theorie) 5-Methansulfonamido-1-(2,6-dichlor-4-trifluormethylphenyl)-pyrazol vom Schmelzpunkt 64-67°C.

nach Verfahren (b):

2 g (4,4 mMol) 5-Bis-(methansulfon)-imido-1-(2,6-dichlor-4-trifluormethylphenyl)-pyrazol werden in einer Mischung aus 5 ml Ethanol und 5 ml konzentrierter Ammoniaklösung suspendiert und 16 Stunden bei Raumtemperatur gerührt; dabei geht die Suspension in Lösung. Das Lösungsmittel wird im Vakuum abdestilliert und der ölige Rückstand in Methylenchlorid und verdünnter Salzsäure aufgenommen. Die organische Phase wird abgetrennt, mit Kochsalzlösung gewaschen und über Magnesiumsulfat getrocknet. Nach Eindampfen im

0192951

Vakuum erhält man 1,5 g (91 % der Theorie) 5-Methansulfonamido-1-(2,6-dichlor-4-trifluormethylphenyl)-pyrazol vom Schmelzpunkt 65-67°C.

Beispiel 3

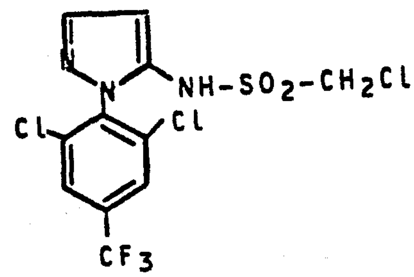

nach Verfahren (a):

10 g (0,034 Mol) 5-Amino-1-(2,6-dichlor-4-trifluormethylphenyl)-pyrazol werden in 35 ml Pyridin gelöst. Bei 0°C bis 5°C werden 21,4 g (0,068 Mol) 47 %iges Chlormethansulfochlorid gelöst in 1,2-Dichlorbenzol zugetropft. Es wird fünf Stunden gerührt, wobei die Temperatur langsam auf 20°C ansteigt. Danach wird der Ansatz in Eiswasser ausgetragen und mit Methylenchlorid extrahiert. Die organische Phase wird abgetrennt, mit verdünnter Salzsäure gewaschen und mit dreimal 100 ml gesättigter Natriumhydrogencarbonat-Lösung extrahiert. Die vereinigten wäßrigen Phasen werden mit 10 %iger Salzsäure auf pH 1 gestellt und das ausgefallene Produkt in Methylenchlorid aufgenommen. Die Methylenchlorid-Phase wird abgetrennt, mit gesättigter Kochsalzlösung gewaschen, über Magnesiumsulfat getrocknet und eingeengt.

Man erhält 8,7 g (63 % der Theorie) 5-Chlormethansulfonamido-1-(2,6-dichlor-4-trifluormethylphenyl)-pyrazol vom Schmelzpunkt 63-66°C.

Beispiel 4

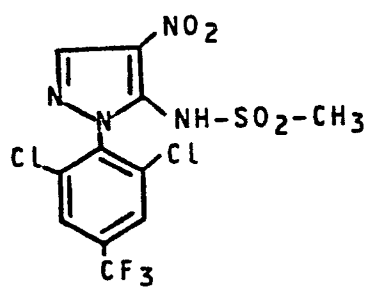

nach Verfahren (c):

3,2 g (8,6 mMol) 5-Methansulfonamido-1-(2,6-dichlor-4-trifluormethylphenyl)-pyrazol werden in 10 ml Eisessig gelöst und bei 10°C bis 15°C nacheinander mit 0,8 ml (8,6 mMol) Essigsäureanhydrid und 0,4 ml (9,3 mMol) 98 %iger Salpetersäure versetzt. Es wird 16 Stunden bei Raumtemperatur gerührt, im Vakuum eingeengt und in 50 ml Methylenchlorid aufgenommen. Diese Lösung wird dreimal mit je 50 ml gesättigter Natriumbicarbonatlösung extrahiert. Die vereinigten wäßrigen Phasen werden mit verdünnter Salzsäure auf pH 1 gestellt, der entstehende Niederschlag wird in Methylenchlorid gelöst und von der wäßrigen Phase getrennt. Es wird mit Kochsalzlösung gewaschen und über Magnesiumsulfat getrocknet. Nach dem Abziehen des Lösungsmittels im Vakuum erhält man 2,8 g (78 % der Theorie) 5-Methansulfonamido-4-nitro-1-(2,6-dichlor-4-trifluormethylphenyl)-pyrazol vom Schmelzpunkt 163°C.

Beispiel 5

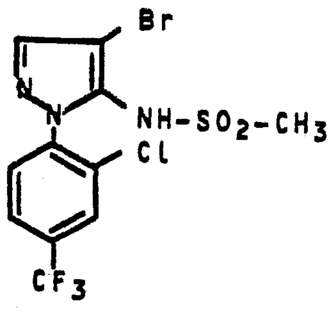

nach Verfahren (c):

3,4 g (0,01 Mol) 5-Methansulfonamido-1-(2-chlor-4-trifluormethylphenyl)-pyrazol werden in 20 ml Methylenchlorid gelöst und mit 0,52 ml (0,01 Mol) Brom, gelöst in 5 ml Methylenchlorid, versetzt. Nach beendeter Zugabe wird eine Stunde nachgerüht, mit 20 ml Methylenchlorid verdünnt und nacheinander mit Bicarbonat-, Thiosulfat- und Kochsalzlösung gewaschen. Die organische Lösung wird dann über Magnesiumsulfat getrocknet und im Vakuum eingeengt.

Man erhält 3,4 g (91 % der Theorie) 4-Brom-5-methansulfonamido-1-(2-chlor-4-trifluormethylphenyl)-pyrazol vom Schmelzpunkt 63-65°C

Beispiel 6

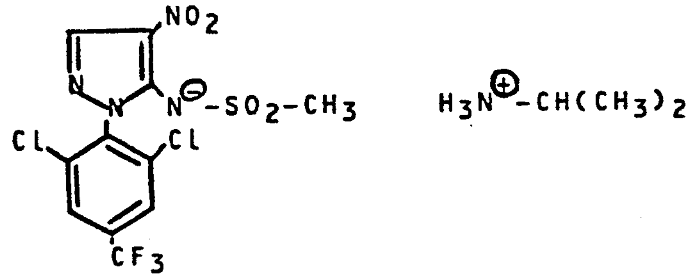

nach Verfahren (d-ß):

3 g (7,2 mMol) 5-Methansulfonamido-4-nitro-1-(2,6-dichlor-4-trifluormethylphenyl)-pyrazol werden in 40 ml Ethanol suspendiert und mit 1,1 ml (10,7 mMol) einer 70 %igen alkoholischen Isopropylaminlösung versetzt. Die entstehende klare Lösung wird im Vakuum eingeengt.

Man erhält 3,4 g (100 % der Theorie) an 5-Methansulfonamido-4-nitro-1-(2,6-dichlor-4-trifluormethylphenyl)-pyrazol Isopropylammoniumsalz vom Schmelzpunkt 188°C.

Beispiel 7

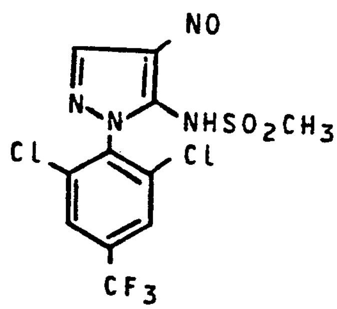

nach Verfahren (c):

1,5 g (4,0 mMol) 5-Methansulfonamido-1-(2,6-dichlor-4-trifluormethylphenyl)-pyrazol werden in 20 ml Ethanol gelöst, auf 0°C bis 5°C abgekühlt und mit 1 ml kaltem Ethylnitrit, sowie 1 ml konzentrierter Salzsäure versetzt. Es wird ca. 6 Stunden bei 0°C bis 5°C und vierzehn Stunden bei Raumtemperatur gerührt. Das Lösungsmittel wird im Vakuum abdestilliert, der Rückstand in 20 ml Methylenchlorid und 10 ml Wasser aufgenommen, die wäßrige Phase mit Natriumacetat-Lösung auf pH 3 gebracht und die organische Phase danach abgetrennt, mit Kochsalzlösung gewaschen und über Magnesiumsulfat getrocknet. Nach dem Entfernen des Lösungsmittels im Vakuum erhält man 1,4 g (87 % der Theorie) 5-Methansulfonamido-4-nitroso-1-(2,6-dichlor-4-trifluormethylphenyl)-pyrazol vom Schmelzpunkt 151°C-153°C.

In entsprechender Weise und gemäß den allgemeinen Herstellungsangaben erhält man die in der folgenden Tabelle aufgeführten 5-Sulfonamido-1-aryl-pyrazole der allgemeinen Formel (I),

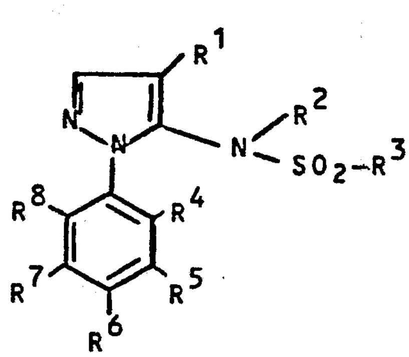

(I)

Tabelle 2

| Bsp Nr | $R^1$ | $R^2$ | $R^3$ | $R^4$ | $R^5$ | $R^6$ | $R^7$ | $R^8$ | Schmelzpunkt(°C) |
|---|---|---|---|---|---|---|---|---|---|
| 8 | H | H | 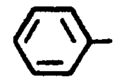 | Cl | H | $CF_3$ | H | H | 150 |
| 9 | H | H | 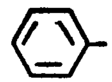 | Cl | H | $CF_3$ | H | Cl | 126 |
| 10 | H | H | $CH_3$ | Cl | H | $CF_3$ | H | H | 137-138 |
| 11 | H | H | $CH_3$ | Cl | Cl | $CF_3$ | H | Cl | Glas<br>IR ($cm^{-1}$): $\nu_{symSO_2}$=1165 |
| 12 | H | H | $CH_3$ | Cl | H | $-SO_2CF_3$ | H | Cl | Glas<br>IR ($cm^{-1}$): $\nu_{symSO_2}$=1165 |
| 13 | H | H | $CH_3-(CH_2)_3-$ | Cl | H | $CF_3$ | H | Cl | Oel<br>IR ($cm^{-1}$): $\nu_{symSO_2}$=1145 |
| 14 | $NO_2$ | H | 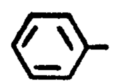 | Cl | H | $CF_3$ | H | Cl | 180-181 |
| 15 | $NO_2$ | H | 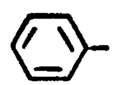 | Cl | H | $CF_3$ | H | H | 150-151 |
| 16 | $NO_2$ | H | $CH_3$ | Cl | H | $CF_3$ | H | H | 64 |

Tabelle 2 (Fortsetzung)

| Bsp. Nr.. | $R^1$ | $R^2$ | $R^3$ | $R^4$ | $R^5$ | $R^6$ | $R^7$ | $R^8$ | Schmelzpunkt (°C) |
|---|---|---|---|---|---|---|---|---|---|
| 17 | $NO_2$ | H | $CH_3$ | Cl | Cl | $CF_3$ | H | Cl | 76 |
| 18 | $NO_2$ | H | $CH_3-(CH_2)_3-$ | Cl | H | $CF_3$ | H | Cl | 155-157 |
| 19 | $NO_2$ | H | $ClCH_2-$ | Cl | H | $CF_3$ | H | Cl | 172-175 |
| 20 | $NO_2$ | H | $CH_3$ | Cl | H | $-SO_2CF_3$ | H | Cl | 209-211 |
| 21 | $NO_2$ | H | $CF_3$ | Cl | H | $CF_3$ | H | Cl | 133 - 138 (Zers.) |
| 22 | H | H | $CF_3$ | Cl | H | $CF_3$ | H | Cl | > 300 |
| 23 | $NO_2$ | $K^{\oplus}$ | $CH_3$ | Cl | H | $CF_3$ | H | Cl | >270 (Zers) |
| 24 | $NO_2$ | $1/2 Mg^{2\oplus}$ | $CH_3$ | Cl | H | $CF_3$ | H | Cl | 150-155 |
| 25 | $NO_2$ | $H_3N^{\oplus}-CH(CH_3)2$ | $CH_3-(CH_2)_3-$ | Cl | H | $CF_3$ | H | Cl | 72 |
| 26 | $NO_2$ | $H_3N^{\oplus}-CH(CH_3)_2$ | $ClCH_2-$ | Cl | H | $CF_3$ | H | Cl | 128-129 |

Tabelle 2 (Fortsetzung)

| Bsp.Nr | $R^1$ | $R^2$ | $R^3$ | $R^4$ | $R^5$ | $R^6$ | $R^7$ | $R^8$ | Schmelzpunkt (°C) |
|---|---|---|---|---|---|---|---|---|---|
| 27 | $NO_2$ | $H_3N^{\oplus}-CH(CH_3)_2$ | $CF_3$ | Cl | H | $CF_3$ | H | Cl | 53 - 60 |
| 28 | $NO_2$ | $H_3N^{\oplus}-CH(CH_3)_2$ | 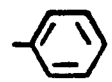 | Cl | H | $CF_3$ | H | Cl | 64-70 (Zers.) |
| 29 | $NO_2$ | $H_3N^{\oplus}-CH(CH_3)_2$ | $CH_3$ | Cl | H | $SO_2CF_3$ | H | Cl | 177-180 |
| 30 | $NO_2$ | $K^{\oplus}$ | $CH_3$ | Cl | Cl | $CF_3$ | H | Cl | >250 |
| 31 | $NO_2$ | 1/2 $Mg^{2\oplus}$ | $CH_3$ | Cl | Cl | $CF_3$ | H | Cl | 147 |
| 32 | $NO_2$ | 1/2 $Zn^{2\oplus}$ | $CH_3$ | Cl | Cl | $CF_3$ | H | Cl | 106-117 |
| 33 | $NO_2$ | $H_3N^{\oplus}-CH(CH_3)_2$ | $CH_3$ | Cl | Cl | $CF_3$ | H | Cl | 105 (Zers.) |
| 34 | $NO_2$ | 1/2 $Mn^{2\oplus}$ | $CH_3$ | Cl | Cl | $CF_3$ | H | Cl | 114 (Zers.) |
| 35 | $NO_2$ | $Na^{\oplus}$ | $CH_3$ | Cl | Cl | $CF_3$ | H | Cl | >250 |

## Anwendungsbeispiele

In den folgenden Anwendungsbeispielen wurde die nachstehend aufgeführte Verbindung als Vergleichssubstanz eingesetzt:

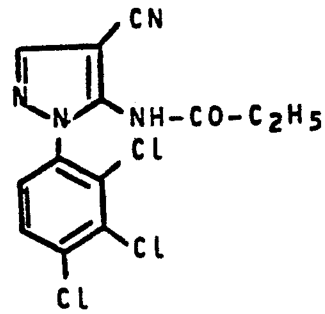

(A)

4-Cyano-5-propionamido-1-(2,3,4-trichlorphenyl)-pyrazol (bekannt aus DE-OS 3 226 513)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test die Verbindungen der folgenden Herstellungsbeispiele: (6), (23)

**Beispiel B**

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der Wirksamkeit sowie in der Nutzpflanzenselektivität gegenüber dem Stand der Technik zeigen in diesem Test z.B. die Verbindungen gemäß folgender Herstellungsbeispiele: (6), (23)

## Patentansprüche

1. 5-Sulfonamido-1-aryl-pyrazole der Formel (I)

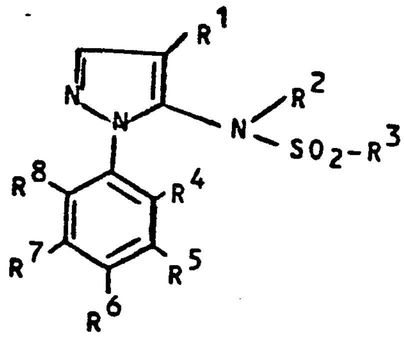

(I)

in welcher

$R^1$ für Wasserstoff, Nitro, Nitroso oder Halogen steht,

$R^2$ für Wasserstoff, Alkyl, für einen Rest $R^3-SO_2-$ oder für ein salzartig gebundenes anorganisches oder organisches Kation steht,

$R^3$ für Alkyl, Alkenyl, Cycloalkyl, Halogenalkyl, Hydroxyalkyl, Alkoxyalkyl oder für jeweils gegebenenfalls substituiertes Aryl steht,

$R^4$ und $R^6$ unabhängig voneinander für Cyano, Nitro, Halogen, Alkyl, Alkoxy, Alkoxycarbonyl, Halogenalkyl, Halogenalkoxy oder für einen Rest $-S(O)_n-R^9$ stehen und

$R^5$, $R^7$ und $R^8$ unabhängig voneinander und von $R^4$ und $R^6$ für die gleichen Reste wie $R^4$ und $R^6$ stehen und zusätzlich für Wasserstoff stehen, wobei

$R^9$ für Alkyl, Halogenalkyl, Amino, Alkylamino oder Dialkylamino steht und

n für eine Zahl 0, 1 oder 2 steht.

2. 5-Sulfonamido-1-aryl-pyrazole der Formel (I)

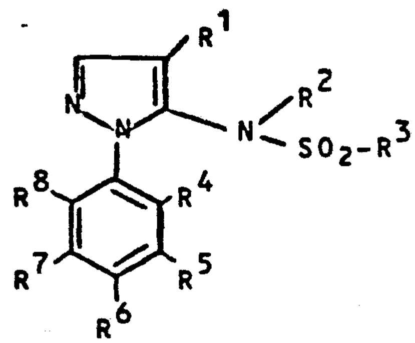

(I)

in welcher

$R^1$ für Wasserstoff, Nitro, Nitroso oder Halogen steht,

$R^2$ für Wasserstoff, geradkettiges oder verzweigtes Alkyl mit 1 bis 8 Kohlenstoffatomen, für einen Rest $R^3$-$SO_2$- oder für ein Äquivalent eines Alkali- oder Erdalkali- oder Übergangsmetallkations oder für ein gegebenenfalls substituiertes Ammoniumion steht,

$R^3$ für jeweils geradkettiges oder verzweigtes Alkyl, Alkenyl, Halogenalkyl, Alkoxyalkyl oder Hydroxyalkyl mit jeweils bis zu 4 Kohlenstoffatomen und gegebenenfalls bis zu 9 gleichen oder verschiedenen Halogenatomen, für Cycloalkyl mit 3 bis 7 Kohlenstoffatomen oder für jeweils gegebenenfalls einfach oder mehrfach, gleich oder verschieden durch Halogen, Cyano, Nitro sowie jeweils niederes Alkyl, Alkoxy, Alkylthio oder Halogenalkyl substituiertes Benzyl oder Phenyl steht,

$R^4$ und $R^6$ unabhängig voneinander für Cyano, Halogen oder für jeweils geradkettiges oder verzweigtes Alkyl, Alkoxy oder Alkoxycarbonyl mit jeweils bis zu 4 Kohlenstoffatomen außerdem für jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder für einen Rest $-S(O)_n-R^9$ stehen, und

$R^5$, $R^7$ und $R^8$ unabhängig voneinander und von $R^4$ und $R^6$ für die gleichen Reste wie $R^4$ und $R^6$ stehen und zusätzlich für Wasserstoff stehen, wobei

$R^9$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

0192951

n für eine Zahl 0, 1 oder 2 steht.

3. 5-Sulfonamido-1-aryl-pyrazole der Formel (I) in welcher

$R^1$ für Wasserstoff, Nitro, Nitroso, Fluor, Chlor, Brom oder Iod steht,

$R^2$ für Wasserstoff, Methyl, Ethyl, n- oder i-Propyl, n-, i-, s- oder t-Butyl, für einen Rest $R^3$-$SO_2$- oder für ein Äquivalent eines Natrium-, Kalium-, Magnesium-, Calcium-, Barium-, Kupfer-, Zink-, Mangan-, Zinn-, Eisen-, Cobalt- oder Nickelions steht, oder für ein gegebenenfalls ein- bis dreifach gleich oder verschieden durch Methyl, Ethyl, n- oder i-Propyl, n-, i-, s- oder t-Butyl oder durch Phenyl substituiertes Ammoniumion steht,

$R^3$ für Methyl, Ethyl, n- oder i-Propyl, n-, i-, s- oder t-Butyl, Allyl, Butenyl, Methoxymethyl, Methoxyethyl, Hydroxymethyl, Hydroxyethyl, Chlormethyl, Dichlormethyl, Trifluormethyl, Cyclopropyl, Cyclopentyl, Cyclohexyl oder für jeweils gegebenenfalls ein- bis dreifach gleich oder verschieden substituiertes Benzyl oder Phenyl steht, wobei als Substituenten infrage kommen: Fluor, Chlor, Brom, Cyano, Nitro, Methyl, Ethyl, Methoxy, Methylthio oder Trifluormethyl,

$R^4$ und $R^6$ unabhängig voneinander für Cyano, Nitro, Fluor, Chlor, Brom, Iod, Methyl, Ethyl, n- und i-Propyl, n-, i-, s- und t-Butyl, für Methoxy, Ethoxy, Methoxycarbonyl, Ethoxycarbonyl, für Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, für Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder für einen Rest $-S(O)_n-R^9$ stehen und

$R^5$, $R^7$ und $R^8$ unabhängig voneinander und von $R^4$ und $R^6$ für die gleichen Reste wie $R^4$ und $R^6$ stehen und zusätzlich für Wasserstoff stehen, wobei

$R^9$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht und

n für eine Zahl 0, 1 oder 2 steht.

0192951

4. **Verfahren zur Herstellung von 5-Sulfonamido-1-aryl-pyrazolen der Formel (I),**

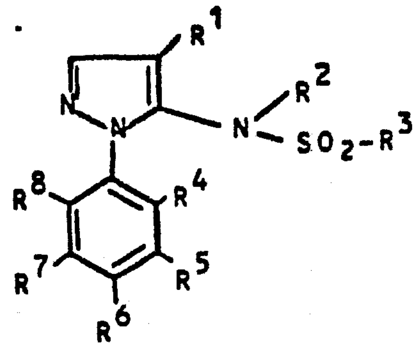

(I)

in welcher

$R^1$ für Wasserstoff, Nitro, Nitroso oder Halogen steht,

$R^2$ für Wasserstoff, Alkyl, für einen Rest $R^3-SO_2-$ oder für ein salzartig gebundenes anorganisches oder organisches Kation steht,

$R^3$ für Alkyl, Alkenyl, Cycloalkyl, Halogenalkyl, Hydroxyalkyl, Alkoxyalkyl oder für jeweils gegebenenfalls substituiertes Aryl steht,

$R^4$ und $R^6$ unabhängig voneinander für Cyano, Nitro, Halogen, Alkyl, Alkoxy, Alkoxycarbonyl, Halogenalkyl, Halogenalkoxy oder für einen Rest $-S(O)_n-R^9$ stehen und

$R^5$, $R^7$ und $R^8$ unabhängig voneinander und von $R^4$ und $R^6$ für die gleichen Reste wie $R^4$ und $R^6$ stehen und zusätzlich für Wasserstoff stehen, wobei

$R^9$ für Alkyl, Halogenalkyl, Amino, Alkylamino oder Dialkylamino steht und

n für eine Zahl 0, 1 oder 2 steht,

dadurch gekennzeichnet, daß man

(a) 5-Aminopyrazole der Formel (II),

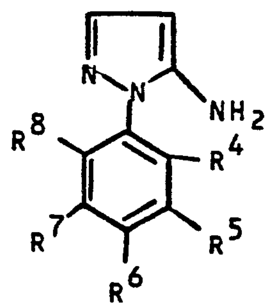

(II)

in welcher

$R^4$, $R^5$, $R^6$, $R^7$ und $R^8$ die oben angegebene Bedeutung haben,

mit Sulfonylchloriden der Formel (III),

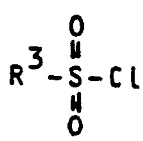

(III)

in welcher

$R^3$ die oben angegebene Bedeutung hat,

gegebenenfalls in Gegenwart eines Verdünnungsmittels und gegebenenfalls in Gegenwart eines Säurebindemittels umsetzt,
oder

(b) die nach Verfahren (a) erhältlichen Bisulfonylamine der Formel (Ia),

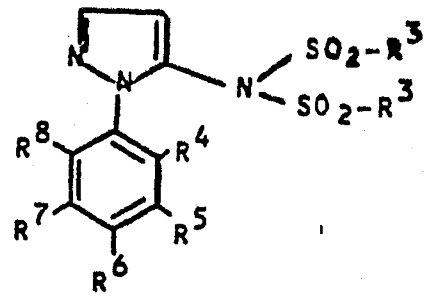

(Ia)

in welcher

$R^3$, $R^4$, $R^5$, $R^6$, $R^7$ und $R^8$ die oben angegebene Bedeutung ahben

mit Basen gegebenenfalls in Gegenwart eines Verdünnungsmittels spaltet,

oder

(c) die nach Verfahren (a) oder (b) erhältlichen 5-Sulfonamido-pyrazole der Formel (Ib),

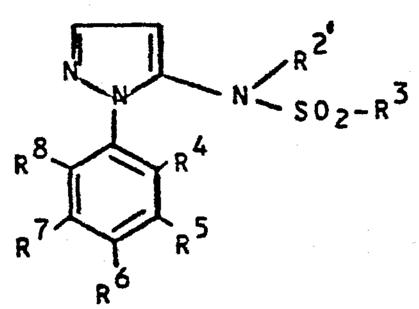

(Ib)

in welcher

$R^{2'}$ für Wasserstoff oder für einen Rest $R^3$-$SO_2$- steht und

$R^3$, $R^4$, $R^5$, $R^6$, $R^7$ und $R^8$ die oben angegebene Bedeutung haben,

mit elektrophilen Agenzien der Formel (IV),

$$R^{1'}\text{-}E \qquad (IV)$$

in welcher

$R^{1'}$ für Halogen, Nitroso oder Nitro steht und

E für eine elektronenanziehende Abgangsgruppierung steht,

gegebenenfalls in Gegenwart eines Verdünnungsmittels und gegebenenfalls in Gegenwart eines Katalysators oder Reaktionshilfsmittels in 4-Stellung substituiert,

oder

(d) die nach Verfahren (a), (b) oder (c) erhältlichen 5-Sulfonamido-pyrazole der Formel (Ic),

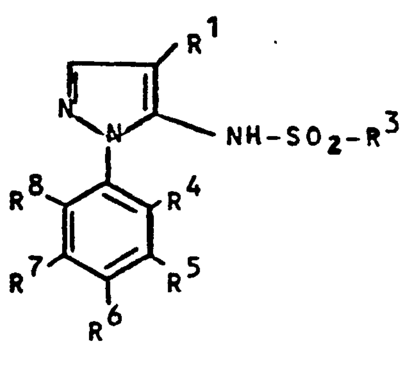

(Ic)

in welcher

$R^1$, $R^3$, $R^4$, $R^5$, $R^6$, $R^7$ und $R^8$ die oben angegebene Bedeutung haben,

entweder

(α) mit Alkylierungsmitteln der Formel (V),

$R^{2''}$-E' (V)

in welcher

$R^{2''}$ für Alkyl steht und
E' für eine elektronenanziehende Abgangsgruppe steht,

gegebenenfalls in Gegenwart eines Verdünnungsmittels und gegebenenfalls in Gegenwart eines Säurebindemittels bzw. Katalysators am Stickstoffatom der Sulfonamidogruppe alkyliert,

oder

(ß) mit Verbindungen der Formel (VI),

$M^{\oplus}$ $G^{\ominus}$ (VI)

in welcher

$M^{\oplus}$ für ein Äquivalent eines anorganischen oder organischen Kations steht und

$G^{\ominus}$ für ein Äquivalent eines geeigneten Gegenions steht,

umsetzt oder mit primären, sekundären oder tertiären Aminen gegebenenfalls in Gegenwart eines Verdünnungsmittels am Stickstoff der Sulfonamidogruppe ein Salz bildet.

5. Herbizide Mittel, gekennzeichnet durch einen Gehalt an mindestens einem 5-Sulfonamido-1-aryl-pyrazol der Formel (I) gemäß den Ansprüchen 1 bis 4.

6. Verfahren zur Bekämpfung von Unkräutern, dadurch gekennzeichnet, daß man 5-Sulfonamido-1-aryl-pyrazole der Formel (I) gemäß den Ansprüchen 1 bis 4 auf Unkräuter und/oder ihren Lebensraum einwirken läßt.

7. Verwendung von 5-Sulfonamido-1-aryl-pyrazolen der Formel (I) gemäß den Ansprüchen 1 bis 4 zur Bekämpfung von unerwünschtem Pflanzenwuchs.

8. Verfahren zur Herstellung von herbiziden Mitteln, dadurch gekennzeichnet, daß man 5-Sulfonamido-1-aryl-pyrazole der Formel (I) gemäß den Ansprüchen 1 bis 4 mit Streckmitteln und/oder oberflächenaktiven Mitteln vermischt.